# EUROPEAN PATENT APPLICATION

(11) **EP 4 289 939 A1**
(43) Date of publication of application: **13.12.2023**
(21) Application number: 22178358.2
(22) Date of filing: 10.06.2022
(51) Int. Cl.: C12N 5/078, A61K 35/17, C12N 5/0783, C12N 15/113, C07K 14/82

(54) **POPULATION OF TRANSFECTED IMMUNE CELLS AND METHOD FOR THEIR PRODUCTION**

(71) Applicant: Apeiron Biologics AG, 1030 Wien (AT); invIOs GmbH, 1030 Wien (AT)
(72) Inventor: DOHNAL, Alexander, 1030 Wien (AT); GUGENBERGER, Romana, 1030 Wien (AT); PEBALL, Bernhard, 1030 Wien (AT); MÜHLEISEN, Hannes, 1030 Wien (AT); SEIDL, Sandra, 1030 Wien (AT); URBAN, Maria, 1030 Wien (AT)
(74) Representative: SONN Patentanwälte GmbH & Co KG

(57) **Abstract**

The invention provides an in-vitro or ex-vivo method for transiently modifying immune cells in a closed processing system comprising the steps: (i) providing immune cells from a biological liquid and/or from a resected tumor from a patient; (ii) purifying the immune cells; (iii) transfecting the purified immune cells with an inhibitory nucleic acid of a immunosuppressive regulator of the immune cells or with a nucleic acid of an immune enhancing factor; (iv) rebuffering the transfected immune cells into a physiological solution; (v) transferring the transfected immune cells into a container; wherein the transitions between steps (i) to (v) are in a closed container system; and a therapy using the transfected immune cells.

## Description

The present invention relates to the field cell preparation and cancer treatment methods.

### Background of the invention

Cancer is the second leading cause of death worldwide and has a major impact on society and healthcare systems across the world. It has been estimated to be responsible for 9.6 million deaths in 2018 (Center for Disease Control and Prevention, 2017; International Agency for Research on Cancer, 2017) and by 2040 the number of new cancer cases per year is estimated to rise to almost 30 million.

Prognosis for patients with advanced disease is poor. Refinements in conventional therapies and the development of novel targeted therapies and immunotherapies have improved outcome for patients with advanced cancers. However, patients with extensive, late-stage disease may have exhausted the available treatment options; in other patients the effects of extensive disease progression and residual side effects from prior treatment regimens may preclude the use of more aggressive therapeutic agents.

The immune system can identify and eliminate tumor cells based on their expression of tumor-specific antigens and other molecules that are either aberrantly expressed or induced by cellular stress. However, most tumor-reactive T cells show low avidity to tumor-associated antigens, which are predominantly "self" proteins. Poor T cell function also reflects deficient expression of costimulatory proteins and/or expression of inhibitory ligands or suppressive cytokines in the tumor microenvironment.

In addition, cancer cell immune editing rationalizes that tumor cell variants arise that can resist, suppress, or avoid elimination by the immune system. In such situations some immune reactivity against the tumor will persist, and treatments able to reinitiate a strong anti-tumor immune response can achieve tumor rejection.

A series of novel cancer immunotherapies have been successfully developed (Murciano-Goroff, Y.R., Warner, A.B. & Wolchok, J.D. The future of cancer immunotherapy: microenvironment-targeting combinations. Cell Res 30, 507-519 (2020). https://doi.org/10.1038/s41422-020-0337-2). These therapies primarily target adaptive immune responses.

One approach is the treatment with monoclonal antibodies. Ipilimumab is a fully human, monoclonal antibody that blocks CTLA-4 a receptor that downregulates the immune system. Ipilimumab disrupts the CTLA-4 inhibition in cytotoxic T cells and boosts the immune response against tumor cells. In a Phase III trial, ipilimumab was the first agent to demonstrate an improvement in overall survival in patients with previously treated, advanced melanoma. The adverse event profile associated with ipilimumab was primarily immune-related (Hoos et al., 2010). Ipilimumab was approved in Europe and in the USA in 2011.

Nivolumab, cemiplimab, pembrolizumab and atezolizumab, avelumab, durvalumab represent a series of clinically approved monoclonal antibodies that disrupt the PD1/ PD-L1 signalling axis. This mode of action lead to enhanced cytotoxicity of T cells against tumor cells showing an improvement of cancer therapies for several tumor types (Murciano-Goroff et al., 2020) .

Sipuleucel-T is an autologous cell-based immunotherapy for prostate cancer in which a patient's white blood cells are stimulated with a fusion protein consisting of the prostatic acid phosphatase antigen and granulocyte-macrophage colony stimulating factor. This fusion protein activates antigen-presenting cells and promotes maturation. The activated blood product is then reinfused, and the activated antigen-presenting cells help the patient's immune system to target prostate cancer cells. Increased inflammatory cytokine production is seen in patients treated with Sipuleucel-T (Kantoff et al., 2010).

A further approach of addressing immune deficiencies preventing an anti-tumor response in vivo is to activate immune cells ex-vivo and then infuse them back into the patient, where they can mount an effective anti-tumor response (e.g. WO 2009/073905 and WO 2012/089736).

Autologous cell therapy is a novel therapeutic intervention in tumor therapy that uses individual's cells, which are manipulated outside the body, and reintroduced into the donor. Advantages of such an approach include the minimization of risks from systemic immunological reactions, bio-incompatibility, and disease transmission associated with grafts or cells not cultivated from the individual.

Most patients with metastatic or recurrent malignancies will develop resistance to currently available treatment options and eventually succumb to their disease.

Hence, there is a substantial unmet need for novel effective and less toxic therapeutic strategies to improve the outcome for patients with advanced late-stage or metastatic malignancies. Object of the present invention is to provide a new cellular therapy to improve the outcome for patients with advanced malignancies.

### Summary of the invention

T-cell based adoptive immunotherapies rely on the delivery of genetically engineered T cells to patients. This methodology has developed enormously over the last decade, extending beyond T cells to other immune cells and becoming one of the most promising therapeutic strategies for treating a range of malignancies.

The present invention provides a platform process with personalized treatment of a variety of solid and liquid tumors. Furthermore, the platform technology is applicable to all kinds of tumors and targets. In particular, the invention works to activate a patient's immune system to raise or increase an immune reaction against these tumors and targets.

The invention provides modification of immune cells (leukocytes) in a closed system, that is aseptically inter-connected. The method comprises three major steps: "MAP I": (initial) purification of immune cells, "MAP II": transfection of immune cells and "MAP III": rebuffering the transfected immune cells with optional further purification.

This process enables automated, closed leukocyte purification (MAP step I) followed by a closed, automatically performed transfection, preferably electroporation, step (MAP step II) to transfect purified autologous leukocytes and a final rebuffering step (MAP step III).

Immune cells are provided, preferably from leukapheresis or from processing a tumor fragment, cells like platelets and erythrocytes are depleted, and the immune cells are modified, preferably by electroporation. Different kinds of nucleic acids can be used for electroporation. The genetically modified cells, preferably electroporated immune cells, are transferred in the final formulation and the immune cells ready for administration ("Drug Product") is received. This process takes about 6 hours.

The cell processing system is a closed container system and the transfection, preferably electroporation, system utilize single-use-tubing sets, which are inter-connected by aseptic welding of the immune cell purification device tubing set to the transfection device tubing set and the tubing set used for the final rebuffering.

In particular, the present invention provides an in-vitro or ex-vivo method for modifying immune cells in a closed processing system comprising the steps: (i) providing immune cells from a biological liquid and/or from a resected tumor from a patient; (ii) purifying the immune cells (MAP step I); (iii) transfecting the purified immune cells with an inhibitory nucleic acid of a immunosuppressive regulator of the immune cells or with a nucleic acid of an immune enhancing factor (MAP step II); (iv) purifying the transfected immune cells and/or rebuffering the transfected immune cells into a physiological solution (MAP step III); (v) transferring the transfected immune cells into a container;
wherein the transitions between each two subsequent steps of steps (i) to (v) are in a closed container system and/or wherein the transition from one step to another step occurs in containers without being opened. The immune cells may be modified transiently.

The transfected immune cells obtained in step (v) can be used as a medicament, in particular to treat cancer.

Checkpoint inhibitor therapy is a form of cancer immunotherapy. The therapy targets immune checkpoints, key regulators of the immune system that when stimulated can dampen the immune response to an immunologic stimulus. Some cancers can protect themselves from attack by stimulating immune checkpoint targets. Checkpoint therapy can block inhibitory checkpoints, restoring immune system function.

Cbl (Casitas B-lineage Lymphoma) proteins are part of a family of ubiquitin ligases involved in cell signalling, protein ubiquitination, and degradation of protein substrates and is one of the immune checkpoint inhibitors. Members of the family include the RING-type E3 ligases c-Cbl, Cbl-b, and Cblc. The E3 ubiquitin ligase Casitas B-lineage lymphoma protein-b (Cbl-b) is a negative regulator of innate and adaptive immunity and a key negative regulator of antitumor immunity. It limits the reactivity of most types of immune cells, particularly lymphocytes and natural killer (NK) cells. Cbl-b is highly expressed in human CD4+ and CD8+ T cells, with expression tightly regulated by TCR, CD28 and CTLA-4 and other co-stimulatory and inhibitory signals. Thus, Cbl-b is involved in tumor-triggered immune evasion and has been described as a "master checkpoint" in immune function, because Cbl-b inhibition simultaneously overrides multiple relevant T-cell suppression pathways e.g. by transforming growth factor β, and immune regulation by both cytotoxic T-lymphocyte-associated protein (CTLA-4) and programmed cell death ligand 1/programmed cell death 1 (PD-L1/PD-1) pathways. Blocking Cbl-b expression upon silencing the Cbl-b gene in immune cells enhances T cell and natural killer (NK) cell activity and results in reduced tumor growth in animal models. Adoptive cellular immunotherapy with Cbl-b silenced murine T-cells significantly inhibited tumor growth in syngeneic mouse models. The Cbl-b silenced murine T-cells were synergistic with anti-PD1, supporting the potential utility of Cbl-b silenced human T-cells in combination with immune checkpoint inhibitors (CPIs).

Immune cells with at least partial Cbl-b inhibition overcome the immune-suppressive tumor microenvironment (TME).

Further immune checkpoint inhibitors are SHP-1 and SHP-2. Src homology 2 domain-containing protein tyrosine phosphatase 1 (SHP-1) is a widely expressed inhibitory protein tyrosine phosphatase (PTP). In T-cells, it is a negative regulator of antigen-dependent activation and proliferation. It is a cytosolic protein, and therefore not amenable to antibody-mediated therapies, but its role in activation and proliferation makes it an attractive target for genetic manipulation in adoptive transfer strategies.

### Detailed description of the invention

Throughout the present disclosure, the articles "a", "an" and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the terms "include" and "including" have the same meaning as the terms "comprise" and "comprising". The terms "comprise" and "comprising" should be interpreted as being "open" transitional terms that permit the inclusion of additional components further to those components that are recited. "Comprising" in connection with a component connected to a range shall mean that further non-recited components are allowed but the recited component linked to that range shall be within said range and not outside said range. The terms "consist" and "consisting of" should be interpreted as being "closed" transitional terms that do not permit the inclusion of additional components other than the recited. The term "consisting essentially of" should be interpreted to be partially closed and allowing the inclusion only of additional components that do not fundamentally alter the nature of the recited subject matter, such as not permitting further non-recited active ingredients but allowing further non-recited auxiliary substances, like buffer components, fillers, and the like.

In the following the term "closed system" or "closed processing system" refers to a system with containers and at least one tubing set that is closed to the outside environment and wherein the processing of cells occurs within the closed system without transferring the cells out of the containers or tubing set of the closed system. Similarly, the term "closed container system" when referring to the transition of the cells between the individual method steps (in particular MAP steps I-III) refers to the means of that transition, i.e. including the container used in that step (e.g. container for purification, container for transfection, container for rebuffering), and optionally transport containers between these steps. A container may be a tube, especially in case of transport container. Optionally, method steps (MAP steps) may also be performed in a tube. Any closed system appropriate for ex-vivo cell treatment methods can be employed with the methods of the present invention. Once a cell sample, e.g., immune cells of blood or of a tumor segment, is obtained and added to the containers of the closed system the cells are not transferred out of the closed system until the modified immune cells are ready to be administered to the patient. At least one in-process control can aseptically be drawn, and/or nucleic acids can aseptically be introduced into the closed system. Thus, the containers of the closed system are not opened to the outside of the environment when the immune cells are transferred from one step to another step of the modification process.

Provided is an ex-vivo or in-vitro method for transiently modifying immune cells, preferably unstimulated rested or activated immune cells, to at least partially delete or reduce an immunosuppressive regulator and/or add immune cell enhancing factors in a closed processing system, and a population of immune cells transfected in a closed system for use in a method for treating cancer.

The present invention describes a novel cellular therapy, especially an ex-vivo method for transiently modifying unstimulated rested or activated immune cells to delete or reduce an immunosuppressive regulator at least partially and/or add immune cell enhancing factors in a closed processing system comprising the steps:
(i)obtaining the immune cells from a biological liquid and/or tumor resected from a patient collected previously;
(ii) purifying the immune cells;
(iii) transfecting the purified immune cells;
(iv) rebuffering the transfected immune cells into a physiological solution;
(v) transferring the transfected immune cells into a container, wherein the transfected immune cells are a therapeutic population of immune cells;
wherein the transition from one step to another step occurs in containers without being opened.

Also provided is an in-vitro or ex-vivo method for modifying immune cells comprising the steps: (i) providing immune cells from a biological liquid and/or from a resected tumor from a patient, thereby providing a cell sample; (ii) purifying the immune cells of the cell sample; (iii) transfecting the purified immune cells with an inhibitory nucleic acid of a immunosuppressive regulator of the immune cells or with a nucleic acid of an immune enhancing factor; (iv) purifying the transfected immune cells and/or rebuffering the transfected immune cells into a physiological solution; (v) transferring the transfected immune cells into a container; wherein the transitions between each two subsequent steps of steps (i) to (v) are in a closed container system. The invention provides a fully closed, automated and thus, real-time and timesaving process without the need for opening the system to the open environment, which provides immune cells for an individualized therapy.

A closed container system means that all containers that contain the cell sample (cell sample container) or treat the cell sample and reagents for the steps (ii), (iii) and (iv) as well as optional excess fluid removal containers are closed and not open. A container for a reagent for step (ii) may be a buffer container, e.g. a erythrocyte lysis buffer container. A container for a cell sample treatment in step (ii) may be a cell purification container. A container for a sample treatment in step (iii) may be a transfection container. A container for a reagent for step (iv) may be a further buffer container. A container for a cell sample treatment in step (iv) may be a further cell purification container. The cell sample container, buffer container, cell purification container, transfection container, further buffer container, further cell purification container, or any combination thereof may be connected with tubes. The connection may be permanently traversable for the cell sample or reagent or intermittently traversable for the cell sample or reagent. An intermittent traversability may contain interruptions in traversability by closing means, such as valves. It is possible to use pairs of closing means, e.g. valves, and by closing both closing means interrupt a connection without opening the system to the environment. Connections can then be reconnected and returning traversability by opening the closing means. Reconnecting connections is preferably sterile or aseptically, i.e. such as by disinfecting temperature treatment, e.g. 120°C or more, as in tube welding. Such closing and opening of closing means, like valves, without exposure of cell sample of the open environment is still considered as embodiment of a closed container system. This allows transport of containers, e.g. between different devices that may be used to perform certain steps, such as a cell purification device, like a leukapheresis device, for step (ii) or an electroporator device for step (iii) or a cell purification device for step (iv). By intermittent interruption of the connections the device of step (ii) may be the same device as for step (iv). Reagents, such as buffers can also be introduced in a closed container system and connected later in the method when required by the individual method steps. The following example for connections may be used:

The method starts with the cell sample. The cell sample may be a sample directly obtained from a patient. Examples are a serum sample, lymphoid fluid sample or tumour fluid sample. It is usually not purified but represents body fluids from the patient. Obtaining the sample from a patient may be a step previous to the inventive method. Usually, the inventive method starts with the sample being in a cell sample container, e.g. a body fluid bag.

The cell sample container (with the cell sample) is connected to a cell purification container. Cell sample is transported through the connection, e.g. a tube, to the cell purification container. The cell purification container is connected to a buffer container. The buffer container contains reagents for method step (ii) that are introduced to the cell purification container in step (ii). The connection may again be a tube. The buffer container is usually a storge bag or flask for holding a buffer. The purification container may be a container suitable for cell purification, such as purification by leukapheresis. An example is a filter container, such as a spinning membrane filtration container or a counterflow centrifugation system. The filter container contains a filter for separating immune cells from impurities in the cell sample. Impurities may be removed from the cell sample in step (ii). Impurities are for example platelets. Also possible for step (ii), in combination or as alternative, is lysis of erythrocytes. Lysed erythrocyte debris can be removed with the filtrations as separated impurities.

The purification container may connect to the transfection container in order to transfer the purified immune cells to said transfection container for step (iii). Transfection may be with an inhibitory nucleic acid or with the nucleic acid of an immune enhancing factor (both referred to as transfection nucleic acid). Examples are siRNA for inhibitory nucleic acid and nucleic acids encoding a T cell receptor against an antigen, e.g. a tumor antigen, as immune enhancing factor. The transfection nucleic acid may be provided in another container, e.g. a syringe. That other container is also connected as closed system to the transfection container or to a transport container for transition between steps (ii) and (iii), e.g. a tube or transport container. The closed system connection is as above, e.g. through the use of closing means and opening the closing means once the containers are attached, thereby avoiding an opening to the open environment.

The transfection container may be in a transfection device. An example for a transfection container may be a tube, e.g. for a flow electroporation.

After transfection, the now transfected immune cells are in a buffer that may still contain transfection nucleic acids and potentially cell debris from immune cells due to cell damage during transfection. The transfection nucleic acids and cell debris are removed during step (iv). Also a rebuffering may occur during step (iv) to produce a physiological buffer that is pharmaceutically acceptable for an reinsertion, e.g. by infusion, to a patient.

The transfection container is connected to the further cell purification container. As above, the connection may be interruptible by closing means, e.g. a pair of closing means, to enable transport in a closed system. The transfected immune cells are transported to the further cell purification container. The further cell purification container may be connected to a further buffer container. As with the buffer container and the purification container for step (ii) also the connection between the further cell purification container may be connected to a further buffer container may be interrupted, e.g. by closing means. These can be opened to allow flow of reagents. The further purification container may be a container suitable for cell purification, such as purification by membrane purification. An example is a filter container, such as a spinning membrane filtration container. The filter container contains a filter for separating immune cells from impurities.

After purification in step (iv) the transfected and purified immune cells are transferred to a container in step (v), e.g. to a drug administration container, such as an infusion bag.

During steps (ii) and (iv) waste fluid may accumulate, e.g. through filtration. This waste fluid may be removed, e.g. transferred to one or more waste containers.

The method represents a rapid, efficient, and scalable process for on-site GMP production and treatment. The cells do not need a cryopreservation at all. No long-lasting manufacturing at central facilities with complex logistic barriers are necessary. The ex-vivo or in-vitro method can be performed decentralized and local manufacturing is possible. Thus, also a shipment of cryopreserved cells can be avoided.

Additionally, flexible adaption of the procedure adaption to physicians' and patient's needs is possible. Hence, a therapy at highest level of individualization can be provided for the tumor patients and especially always the current tumor status of the patient is treated. Tumor cells have the tendency to escape recognition by the immune system. using the current immune cells of a patient for the method of invention has the advantage that a therapy with modified autologous immune cells, which is currently necessary, can be provided for the patient that reflects the current immune response against the tumor.

Preferably, the immune cells are mononuclear or polymorphonuclear immune cells, preferably peripheral blood mononuclear cells (PBMCs), preferably peripheral blood lymphocytes (PBLs), natural killer (NK) cells, T cells, B cells, monocytes, and/or tumor infiltrating lymphocytes (TILs) from solid tumors or tumor associated fluids. It is also advantageous that the most recent patients' blood cells are used for the method and production for the population of immune cells of the invention, which are provided as Drug Product.

The term immune cells as used herein refers to a population of cells comprising, essentially consisting of, or consisting of one or more types of immune cells. In preferred embodiments, the immune cells are blood immune cells or immune cells from solid tumors. One or more types of immune cells are selected from neutrophils, antigen-presenting cells, PBMCs, granulocytes, leukocytes, lymphocytes (T cells, B cells, NK cells, innate lymphoid cells), monocytes, macrophages and/or dendritic cells. In a preferred embodiment the immune cells are autologous immune cells. Also, an army of various immune cells can be used for transient silencing in the closed processing system for the treatment of cancer.

In accordance with the invention the mononuclear cells are transfected transiently. A transient transfection in the context of the invention means that a nucleic acid molecule (inhibitory nucleic acid and/or the nucleic acid of an immune enhancing factor) is introduced into the cell but does not integrate into the genome of the cell. As such, the introduced nucleic acid molecule is not able of being inherited by the progeny thereof.

The population of immune cells are mononuclear or polymorphonuclear cells, preferably peripheral blood mononuclear cells (PBMCs) and may be obtained from peripheral blood, from bone marrow, spinal fluid, cerebrospinal fluid and/or from tumor tissue of the subject (patient) as collected previously.

In another embodiment of the invention the immune cells can be tumor infiltrating cells (TILs), that may be obtained from single cell preparations of primary tumors and/or metastasis and/or spinal or cerebrospinal fluid.

In a further preferred embodiment, the immune cells, preferably the PBMCs, are obtained by apheresis from a patient, preferably by a single standard leukapheresis procedure and the tumor infiltrating cells are obtained from processed tumor fragments or tumor associated liquids. The leukapheresis product from a respective patient is received in a container, preferably in an out-put bag from leukapheresis. The leukapheresis sample contains a target cell number of approximately 1×10⁶ to 1×10¹⁰ PBMCs/kg, preferably 1.2×10⁸ PBMCs/kg in approximately 100 to 500 mL, preferably 200 mL in the cell sample. In respect of leukapheresis a high volume of PBMCs is available. PBMCs/kg refers to the number of PBMCs per kg body weight of the patient (subject).

Accordingly, the immune cells comprise, essentially consist of, or consist of one or more leukapheresis products, or processed tumor fragments, or one or more subsets thereof, containing e.g., PBMCs or leukocytes. The immune cell preparation may further comprise other cells, e.g., non-immune cells, such as erythrocytes or platelets.

In preferred embodiments, the immune cells are autologous immune cells comprising, essentially consisting of, or consisting of specific immune cell types, e.g., T cells and/or NK cells.

The cells of the e.g., leukapheresis product containing PBMCs can be further processed, e.g., to enrich certain immune cell types, e.g., by depleting other cell types, by specific stimulation e.g., with tumor antigens or tumor cells from the subject, or by isolating certain immune cell types from the population of immune cells.

In a preferred embodiment a first in-process control is aseptically drawn from apheresis out-put material or from tumor resected material previously collected from patients. It is possible to measure the white blood cells and platelets quantity and haematocrit by complete blood count as well as to test for sterility. Aseptically drawing the control sample does not open the system to the environment. It can be through a closed connection, e.g. with closing means at connection means (e.g. tubes or needles), these closing means being in closed configuration when the connection means are connected to containers, with the connecting step being aseptic or sterile, e.g. by disinfecting, such as by temperature elevation, e.g. 120°C or more, such as by a tube welder. Another option is a connection, e.g. through a membrane or filter valve.

The immune cells of the cell sample can be purified, especially washed, preferably with a phosphate buffered saline EDTA solution to eliminate cellular debris. The washing step can be performed in several cycles.

During the first process step, MAP step I (method step (ii), the platform method allows red blood cell (RBC) lysis and platelet (PLT) depletion, preferably automated, if necessary, followed by rebuffering of leukocytes into electroporation (EP) buffer for the following MAP step II (method step (iii)).

Before starting with leukocyte purification of MAP step I, buffer bags and a leukocyte collection bag are aseptically connected to the respective ports of the single-use tubing set, which subsequently is mounted onto the cell processing device.

After entering the required parameters and completion of the device set-up and priming procedures, the starting material containing leukocytes is aseptically connected to the input port of the tubing set. Depending on the cell processing device used, the entire content or aliquots of starting material are automatically transferred into the tubing set to run MAP step I. A successful completion of the cell processing run recorded by an internal device monitoring system is reported for batch release.

In the detailed process of MAP step I, several wash cycles deplete smaller-sized cells and cell debris from the starting material, which enables PLT depletion, while RBCs are removed by an incubation cycle using a lysis. The lysis might be performed before or after loading of the starting material. Following the RBC lysis leukocytes are washed at least once in a wash cycle and rebuffered into the respective electroporation buffer. The entire MAP step I procedure takes ≤ 2 hour to collect ≤ 200 mL leukocyte suspension resuspended in electroporation buffer.

In a preferred embodiment of the invention 6 cycles of PBMC purification are performed. In cycles 1 to 3 the red blood cells are lysed and platelets are depleted and in cycles 4 to 6 further platelets are depleted and the cells are rebuffered in transfection, preferably electroporation, buffer.

Preferably in a first cycle the cell sample, preferably PBMCs, are washed with PBS/EDTA solution.

After and/or during purification of the immune cells, preferably in the second cycle, the red blood cells (RBC) of the cell sample are lysed, preferably by using a lysis buffer with a pH value of 7 to 8, preferably with a pH value of 7.4, containing chloride, ammonium, potassium and/or bicarbonate ions, preferably an ammonium chloride potassium lysis buffer, and the platelets are depleted. When using a lysis buffer as mentioned above only erythrocytes are lysed whereas all other cells, especially the immune cells of the sample, are not damaged and remain viable.

In some embodiments of the invention the immune cells of the cell sample can be purified and washed again, preferably in the third cycle, and thereby platelets and cell debris are depleted, preferably using a spinning-membrane technology, elutriation and/or counter-flow centrifugation. Using the spinning-membrane technology, preferably the filter membrane of the spinning column has a pore size of less than 4 µm.

Further on, the lysis of the red blood cells and the platelet depletion can be followed by rebuffering of the immune cells into transfection buffer to result in a cell sample solution in the container that can be used for further proceeding, preferably in cycles 4 to 6. During these cycles also further cell debris, especially platelets, can be depleted.

At the end of the purification and rebuffering procedure, leukocytes resuspended in transfection, preferably electroporation, buffer are transported, preferably pumped, into the leukocyte collection bag, which is then aseptically reconnected to the transfection, preferably electroporation, device tubing set to directly continue with MAP step II including leukocyte transfection via electroporation.

Preferably, at this point, the immune cells in the cell sample solution are cooled prior to the modification, preferably prior to electroporation. The leukocyte bag might be stored in a refrigerator at 2°C to 8°C for at least 10 to 15 minutes for pre-cooling until proceeding with MAP step II.

In accordance with the invention, after the purification and resuspension of the immune cells a second in-process control can aseptically be drawn from the cell bag containing purified and resuspended mononuclear cells in the cell sample solution to determine activity and potency of e.g. immune checkpoint inhibition, especially to measure the default status of immune cell activity regarding the expression of the transfected gene and potency, e.g., regarding IL-2 production when the cells are cbl-b siRNA transfected.

The modification, especially the transfection, of immune cells in the cell sample can be performed by microinjection, liposomes, electroporation, particle gun, magnet-assisted transfection, sonoporation, nanoparticles and/or cell squeezing technology.

Using an automated, closed electroporation system, high numbers of leukocytes can be processed by electroporation, preferably consecutive flow electroporation.

In an alternative embodiment of the invention the immune cells can also be electroporated in a single shot cuvette in the closed system.

In a preferred embodiment of the invention, the modification of the immune cells is performed by flow electroporation with siRNA directed against at least one immune checkpoint inhibitor. Flow electroporation technology uses an electrical charge to produce the reversible permeability of cell membranes. This allows for the transfer of molecules, such as nucleic acids, into the cells thereby creating cell therapies. In an alternative embodiment of the invention the electroporation of the immune cells can be performed in a large volume cuvette.

The transfection, preferably the electroporation, of the immune cells is performed with at least one nucleic acid, preferably DNA, mRNA or siRNA, and/or peptide nucleic acids (PNAs) which causes modification of the expression pattern of one or more immune checkpoint inhibitor genes, chemokine receptors, cytokines, and/or chimeric antigen receptors.

The term "nucleic acid" as used herein refers to nucleic acids that are substantially free of other macromolecules, such as lipids, polymers, and proteins. The nucleic acid, such as a self-replicating RNA, is not formulated with other macromolecules to improve cellular uptake. Accordingly, the nucleic acid is not encapsulated in, absorbed on, or bound to a liposome, a microparticle or nanoparticle, a cationic emulsion, and the like.

An alternative form of RNA, termed small interfering or 'siRNA', could affect cells differently to mRNA, blocking normal protein production instead of triggering it. There are two forms of siRNA treatment: antisense oligonucleotides (ASO) and RNA interference (RNAi). RNA silencing is a conserved biological response to double-stranded RNA molecules thus regulating gene expression. ASO and RNAi are short sequences of RNA capable to specifically bind to a target sequence and to block normal protein production.

In RNA interference technology, siRNAs bind to the RNA-induced silencing complex ("RISC"), where one strand (the "passenger strand" or "sense strand") is displaced and the remaining strand (the "guide strand" or "antisense strand") cooperates with the RISC to bind a complementary RNA (the target RNA). Once bound, the target RNA is cleaved by the RNA endonuclease Argonaute (AGO) in the RISC and then further degraded by RNA exonucleases.

Key challenges in the development of oligonucleotide therapeutics, e.g., siRNA therapeutics, include (i) poor stability of the compounds, (ii) low efficiency of in vivo delivery to target cells, and (iii) side effects such as "off target" gene silencing and unintended immunostimulation. Among these, the most significant obstacle is the targeted delivery and subsequent cellular uptake of RNAs. To overcome some of these obstacles, various chemical modifications of the oligonucleotide, including (i) sugar modifications, (ii) internucleotide linkage modifications, and (iii) nucleobase modifications are attempted. While these chemical modifications lead to enhanced stability and reduced immunogenicity of RNAs, these modifications are still insufficient to deliver these large, negatively charged macromolecules across the negatively charged phospholipid bilayer of the cell membrane and into the cytoplasm.

As it comes to siRNA used as a therapeutic approach, structural modifications are crucial to stabilize the molecule and prevent it from destruction. A hallmark of RNA silencing is a class of short RNAs which are processed from double-stranded precursor by the enzyme Dicer.

Various approaches are being used to treat cancers including both mRNA vaccines against cancer associated antigens and siRNA silencing cancer genes. The siRNA approach was demonstrated to prevent cancer growth in a 2010 clinical trial (Davis ME, Zuckerman JE, Choi CH, Seligson D, Tolcher A, Alabi CA, Yen Y, Heidel JD, Ribas A. Evidence of RNAi in humans from systemically administered siRNA via targeted nanoparticles. Nature. 2010 Apr 15;464(7291):1067-70.).

Genetic modifications with siRNA are transient and contrary to other cell therapies, where a long-term persistence of genetically modified cells is observed.

After discovery of RNA interference research focused on the opportunity to actively interfere with the production of target proteins by using small (approximately 22 nucleotides) siRNA molecules. Another challenge arises by making those siRNAs stable and resistant against degradation by enzymes. siRNAs offer the advantage for use in a therapeutic approach that they can easily be brought into cells by making the cells permeable. Transient permeability can be achieved through squeezing, electroporation, or chemically etc. siRNAs are designed to specifically bind to their target sequence thus preventing other genes from silencing ("off-target" effects), a phenomenon often seen with other cancer therapies. Despite a transient inhibition of the target gene, which minimizes safety concerns, RNAi holds promise for the treatment of diseases in many different fields, including Immuno-Oncology. In this context siRNA-silencing of novel immune checkpoints such as Cbl-b reactivate the antitumor immune response and trigger destruction of cancerous cells.

The siRNA of the present invention targets Cbl-b, wherein the siRNA is selected from the group consisting of various siRNA sequences targeting Cbl-b.

In a preferred embodiment at least a gene or a part of a gene of one or more immune checkpoint inhibitors is silenced or reduced and/or at least a gene or a part of a gene of one or more chemokine receptors, cytokines, and/or chimeric antigen receptors or combinations thereof are inserted in at least a portion of the population of transfected immune cells.

Such transient transfection leads to transient target gene silencing, since neither the siRNA nor the mRNA molecule is stably integrated into the target cell genome and are degraded after a certain time, so that the target gene expression will resume.

After sample withdrawal for process control, a syringe containing the nucleic acid payload, siRNa or mRNA or DNA, is connected to the leukocyte bag and the payload is transferred into the leukocyte bag for subsequent electroporation.

After payload transfer, the electroporation device tubing set connected to the leukocyte collection bag is mounted onto the electroporation device to run the closed, automated electroporation.

Consecutive electroporations are performed automatically to process the leukocyte suspension in a time frame of approximately one minute per electroporation.

The drug substance (DS), which is defined as autologous leukocytes transfected with the payload, is collected in a leukocyte bag for further processing.

The DS is further processed after an optional holding time depending on the device of 30 to 60 minutes at room temperature to generate the drug product (DP) by aseptically reconnecting the leukocyte bag, containing DS, to the cell processing device tubing set used for final formulation. No defined holding or storage time of DS applies.

The processing routines of the cell processing device and the electroporation device are programmed by the respective equipment manufacturer or during method development. All programs are access-controlled, and an audit trail is available for traceability of changes.

In some embodiments of the invention said one or more immune checkpoint genes is/are selected from the group comprising Cbl-b, PD1, PD-L1, PD-L2, CASP, CTLA4, FoxP3, LAG-3, TIM-3, TIGIT, A2AR, KIR, BTLA, VISTA, B7-H3, B7-H4, SHIP, SHP-1, SHP-2, IL-1R8, NKG2A,CD96, CD112R, IDO, IRG-1, STAT-3, JAKs, Arg-1, Nos-2, Cish, TGFb, PKA, TNFRSF, preferably coding for the human Cbl-b and/or SHIP gene, or a combination thereof. The target nucleic acid sequence can be the sequence of a further immunosuppressive regulator, chemokine receptor and/or cytokine or a combination thereof. Any of these immune checkpoint genes, immunosuppressive regulator, chemokine receptor can be targeted by the transfection nucleic acid, especially the inhibitory nucleic acid.

In a further embodiment of the invention the transfected nucleic acid is coding for at least one gene selected from a group comprising TMEM222, SOCS1, CDKN1, CD5, UBASH3A, RASA2, CD5, TNFAIP3, TCEB2, NR2F6, TET2, BiTEs, or a combination thereof. These are examples for nucleic acids of immune enhancing factors.

The invention may further include CAR-T cells engineered by mRNA transfection, for example expressing chimeric antigen receptors (CARs) against EGFR, NY-ESO, CEA, GD2 for transient protein expression.

Blockade of Cbl-b function thereby activates both adaptive and innate anti-tumor mechanisms leading to more effective tumor destruction. Cbl-b silenced PBMCs represent a novel approach with the concept of enhancing anti-tumor immune responses leading to an improvement in cancer therapy.

Cbl-b is known as a master immune checkpoint, that regulates various immune pathways in a cell type-dependent manner.

Modulating Cbl-b is central in fighting tumor cells in a broad range of tumor indications. Cbl-b acts a negative regulator and major signalling hub for the activation of tumor-specific immune cells. Cbl-b can be silenced through binding of siRNA to Cbl-b mRNA. The Cbl-b deficiency activates a variety of immune cells, like helper T cells, cytotoxic T cells, B cells, and NK cells for tumor destruction.

The following section describes the role of Cbl-b in the context of anti-tumor versus autoimmunity. The Cbl-b gene encodes a member of E3 ubiquitin ligases that mediate ubiquitin attachment to various secondary signalling molecules within immune cells. Along these lines, Cbl-b regulates various immune-stimulatory pathways in a cell type-dependent manner and, as a result, has been described as a 'master checkpoint' of immune function.

Cbl-b plays a role in both the adaptive (T and B cells) and the innate (natural killer (NK) cells and monocytes) immune system. Blocking its activity in T cells among other immune cell subtypes can therefore effectively enhance both tumor antigen-specific activity and recognition of general tumor cell features.

In T cells E3 ubiquitin ligase activity restricts cell activation and can promote anergy (unresponsiveness of T-cells against self-antigens, Schmitz, 2009). Attachment of ubiquitin to secondary signalling molecules disrupts T cell activation via the major histocompatibility complex (MHC)/T cell receptor (TCR). A deficiency in Cbl-b uncouples CD3+ T cells from the requirement for CD28 co-stimulation for downstream activation, establishing an active role for Cbl-b in the induction and maintenance of peripheral T cell tolerance (Jeon et al., 2004). Silencing of Cbl-b, therefore, promotes T-cell activation.

Tumors lack the costimulatory proteins that bind CD28, which helps to minimize T cell activation; in addition, CD28 expression is often lost on T cells that have differentiated into effector cells exposed to repetitive stimulation (Topp et al., 2003). Disrupting Cbl-b expression in such tumor-reactive T cells may provide a unique strategy for increasing autocrine interleukin-2 (IL-2) production, to promote proliferation and survival following tumor recognition in the absence of costimulatory molecules. This approach may also reduce the threshold for T cell activation, allowing low-avidity T cells to respond to tumors expressing small amounts of antigen.

In a preferred embodiment of the invention the cells are transiently transfected with the Cbl-b siRNA molecule by electroporation. Cbl-b checkpoint inhibitor has distinctive advantages over traditional checkpoint blockades. As other checkpoint inhibitors act mostly on T cells Cbl-b inhibitors show universal action according to reactivation of various cell types for antitumor immunity: CD4+ T cells, CD8+ T cells, NK, NKT, B cells, antigen presenting cells (APC).

The Drug Product comprising silenced Cbl-b cells can be used as monotherapy as well as in combination with other immune checkpoint inhibitors or other anti-tumoral therapies.

The siRNA and mRNA of the any immune checkpoint inhibitor can be used in a concentration of 1.0 to 10 mg/mL, preferably 5.0 mg/mL in phosphate-buffered saline for electroporation.

The siRNA is injected, preferably with a syringe, into the container with the cell sample solution.

In accordance with the invention the siRNA is injected through a 0.2 µm filter connected to the swabable Luer second in-process control withdrawal port thereby assuring a closed manufacturing process and avoiding the insertion of particles.

In some embodiments of the invention the immune cells in the transfection buffer, preferably in electroporation buffer, in combination with siRNA are processed in multiple electroporation cycles in an automated electroporation device.

Preferably each electroporation cycle is in a time frame of 10 seconds to 5 minutes, preferably 30 seconds to 2 minutes, most preferably one minute.

The mRNA silencing efficacy of transfected mononuclear cells is more than 15 % in relation to untreated mononuclear cells.

In the final rebuffering process step (step iv), further depletion of residual undesired cells and cell debris is performed followed by rebuffering the leukocytes into the final solution for administration, preferably 0.9% NaCl solution containing stabilizing agent like albumin or a carbohydrate source. Furthermore, electroporation buffer as the Drug Substance matrix and excess free payload are strongly reduced.

The collected leukocyte suspension is subsequently adjusted to the target volume containing the defined cell number for infusion.

The purified transfected immune cells (Drug Product) may be stored and/or can be infused from the bag directly detached after the last manufacturing step.

An aliquot of Drug Product is drawn aseptically for immediate release testing. Upon removal of this aliquot the remaining volume defines the maximum manufacturable dose (MMD) of leukocytes for potential infusion.

In a final step (v) the immune cells can be transferred into a container, preferably an infusion bag to receive the Drug Product. Preferably the autologous transfected immune cells are rebuffered into the final formulation, which is a physiological formulation with a stabilizing agent, preferably into 0.9% NaCl solution containing 1% to 4 % autologous human serum, preferably 0.9% NaCl containing 0.7 g/l to 4.5 g/l human serum albumin for improving viability and stability of the Drug Product.

In parallel to the rebuffering step immune cells are purified and further depleted from residual platelets and cell debris, preferably using the spinning-membrane technology and/or elutriation. Furthermore, electroporation buffer and excess siRNA are reduced to receive the therapeutic population of immune cells for the final Drug Product.

In a preferred embodiment of the invention the concentration of cells in the final solution is about 5×10⁵ to 5×10¹⁰, preferably 5×10⁶ to 5×10⁷ immune cells/mL.

Most preferably there are transfected cells in the final solution at a concentration of 5×10⁵ to 5×10⁶ up to 5×10⁷ mononuclear cells/mL and build the Drug Product.

Preferably the Drug Product contains more than 40 % of mononuclear cells among leucocytes.

It is also possible that the transient modified immune cells of the final formulation are collected directly in an infusion bag, whereas cell aggregates larger than 200 µm are filtered by a membrane, hence cell aggregates larger than 200 µm do not attain to the final infusion bag. This membrane improves product analysis because the samples taken do not contain any inhomogeneities and the visual inspection of the drug product shows only very small aggregates at most, even towards the end of the shelf life.

In accordance with the invention the at least one container, preferably the bag with immune cells and/or the at least one buffer container, the at least one tubing set connecting the containers, and/or the at least one withdrawal-port, preferably for in-process controls, is/are aseptically interconnected using a sterile tubing welder.

In accordance with the invention the immune cells can be expanded in the closed system prior and/or post transfection, preferably electroporation, of the immune cells to obtain a larger number of cells. Usually, unstimulated cells are used for the present invention. But e.g., when tumor infiltrating cells (TILs) are used for performing the method of invention these cells will have to be expanded and therefore could be stimulated or activated. The expansion of immune cells could either be performed before the transfection of immune cells or after transfection of immune cells or before and after transfection of immune cells.

The invention also relates to a population of immune cells for use in a method for treating cancer, wherein the population of immune cells is obtainable by a method of the invention. E.g. a method performed in a closed system comprising:
(i)obtaining immune cells from a biological liquid and/or tumor resected from a patient collected previously;
(ii) purifying the immune cells;
(iii) transfecting the purified immune cells;
(iv) rebuffering the transfected immune cells into a physiological solution;
(v) transferring the transfected immune cells into a container, wherein the transfected immune cells are a therapeutic population of immune cells;
wherein the transition from one step to another step occurs in containers without being opened or in a closed container system. The population of immune cells (Drug Product) can be generated and tested for re-infusion to the patient in about 5 hours, whereas obtaining of cells, preferably the leukapheresis, is not included within these 5 hours. It is administered as a freshly manufactured cell suspension, neither cryo-preserved nor ex-vivo or in-vitro expanded, and has to be re-infused to the patient before decrease of cell viability and potency. Additionally, rapid release testing allows Drug Product re-infusion within 2 hours after finalizing Drug Product manufacturing.

In a preferred embodiment of the invention more than 30 % of the immune cells of the biological sample, preferably of NK cells, T cells and B cells contain siRNA, mRNA or DNA after transfection, preferably after electroporation.

The population of immune cells of this embodiment is for use in autologous cell therapy using at least one immune checkpoint inhibitor selected from a group comprising Cbl-b, PD-1, PD-L1, PD-L2, CASP, CTLA4, FoxP3, LAG-3, TIM-3, TIGIT, A2AR, KIR, BTLA, VISTA, B7-H3, B7-H4, SHIP, SHP-1, SHP-2, IL-1R8, NKG2A,CD96, CD112R, CD160, CD244, IDO, IRG-1, STAT-3, JAKs, Arg-1, Nos-2, Cish, TGFb, PKA, TNFRSF, or a combination thereof to treat various cancers.

The population of immune cells is used in the treatment of cancer.

Preferably, the population of immune cells is provided for use in the treatment of leukaemia, head and neck cancer, Glioblastoma multiforme (GBM), lung cancer, colon cancer, pancreas cancer. At least all solid and haematological tumors in early as well as in advanced lines of therapy can be treated.

The invention further describes a method for treatment or prophylaxis of tumor disease in a subject administering a population of immune cells.

The population of immune cells is administered in at least one dose and one dose to be administered to the patient comprises 5×10⁶ to 5×10¹⁰, preferably 5×10⁷ to 5×10⁸, transfected PBMCs/kg or TILs/kg, so that due to systemic and/or intratumoral and/or intranodal re-infusion re-educated immune cells travel in the blood to find tumor cells. /kg refers to per kg body weight of the patient.

In accordance with the invention the patients receive intravenously or intratumorally or intranodally a multiple dosing of a population of immune cells, preferably one to ten, preferably three to five, and modified immune cell infusions are administered at intervals of 2 to 10 weeks, preferably 3 to 7 weeks, most preferably at 4 weeks for at least 2, preferably 3 cycles.

The population of transfected immune cells produced according to the method of the invention is preferably used in the treatment of tumour diseases but can also be used for prophylaxis in a special patient cohort.

The Drug Product comprises autologous human PBMCs transfected ex-vivo or in-vitro with small interfering ribonucleic acid that silences Cbl-b messenger ribonucleic acid. Cbl-b -silenced PBMCs are reinfused back to the patient, where they can respond to tumor antigen stimulation with enhanced proliferation and cytokine production. Cbl-b - silenced cytotoxic T-cells and NK cells can directly mediate tumor cell killing. Cbl-b -silenced B and mononuclear cells can serve as antigen-presenting cells and elicit more vigorous immune responses against tumor antigens.

The cell therapy of the invention has various advantageous attributes versus other cell therapies. The genetic modification is transient, solid as well as liquid tumors can be treated, and innate and adaptive immune cells are activated.

The manufacturing process is affordable, in real-time and more scalable and not expensive and time-consuming.

The treatment with modified immune cells is safe and well tolerated and do not show any toxicities.

The leukapheresis site can be the manufacturing site as well as the clinical treatment site. The cell sample do not have to be transported.

The flexibility of RNAi technology could expand the applicability of cell therapy to additional immune checkpoints and holds potential for new therapeutic approaches.

The present invention is further illustrated by the following figures and examples, without being limited to these embodiments of the invention.

### Figures:

**Figure 1****.** sfFACS data demonstrating composition of Drug Product;
**Figure 2****.** sfFACS data illustrating characterization of Drug Product;
**Figure 3****.** sfFACS data illustrating impurities of Drug Product;
**Figure 4****.** Flow Chart of fully closed manufacturing process for the generation of Drug Product;
**Figure 5****.** Immune subtype distribution in Cbl-b siRNA electroporated PBMCs;
**Figure 6****.** Screening for the selection of optimized siRNAs for Cbl-b silencing in PBMCs;
**Figure 7****.** Cytokine induction as parameter for Cbl-b silencing efficiency in PBMCs;
**Figure 8****.** IL-2 fold increase comparing Drug Product to no pulse control measured by ELISA;
**Figure 9****.** Percentage of Cbl-b RNA expression of drug product in relation to no pulse control;
**Figure 10****.** Percentage of Cbl-b Silencing comparing of Drug Product and no pulse control measured with qPCR;
**Figure 11****.** Percentage of Cbl-b silencing on protein levels comparing DP and NPC using WB analysis;
**Figure 12****.** Cbl-b quantification in cell lysates of DP and NPC using LC-MS/MS;
**Figure 13****.** IFN-γ production in response to stimulation with peptides;
**Figure 14****.** eGFP fluorescence in leukocytes transduced with eGFP-mRNA quantified by flow cytometry.

### Experiments and Examples

### Human Peripheral Blood Mononuclear Cell Experiments

In humans Cbl-b deficiency enhances anti-tumor activity of T and NK cells whereby the expression of inflammatory cytokines and activation marker upon T cell receptor (TCR) stimulation as well as the proliferation and anti-tumor cytotoxicity are enhanced. Additionally, T cells are resistant to TGF-β mediated immune suppression. The activation of NK cells is increased upon cytokine stimulation or tumor cell contact.

Primary human T-cells were isolated from Cbl-b silenced PBMCs by magnetic enrichment and stimulated with anti-TCR and anti-cluster of differentiation CD28 agonistic antibodies (anti-CD3/CD28 stimulation). Treatment resulted in enhanced production of IFN-γ and IL-2 by T-cells. In addition, Cbl-b silencing in human T-cells also induced increased secretion of IL-12 and IL-17.

Primary human T-cells were isolated from PBMCs, labelled with carboxyfluorescein succinimidyl ester (CFSE; an indicator of proliferation) and transfected either with control or Cbl-b siRNA. The resulting cells were stimulated with anti-CD3/CD28 antibodies and CFSE levels showed a higher level of cell counts at lower CFSE concentrations, indicative of cell proliferation reducing the level of dye in daughter cells. Neither cytokine production nor cell proliferation was induced in unstimulated T-cells showing that Cbl-b silencing enhances T-cell activities only in the context of antigen-stimulation and does not lead to a general systemic activation of all T-cells.

Transient transfection of PBMCs with Cbl-b siRNA resulted in efficient Cbl-b depletion from both T-cells and NK cells. The PMBCs transfected with Cbl-b siRNA were incubated for 4 hours with SKBR3 breast cancer cells in the presence or absence of an antibody recognizing Her2 and IL-2 and IL-12 (NK cell-stimulating cytokines). Though PBMCs are generally very poor effector cells in cytotoxicity assays, Cbl-b silenced PBMCs showed enhanced SKBR3 cell killing in the presence of anti-Her2 antibody, IL-2 and IL-12.

### Animal Tumor Experiments

In a mouse model it could be shown that Cbl-b deficiency protects mice in various tumor models whereby the tumor antigen-specific T cells mediate a long-term protection, the hyperreactive NK cells kill primary and metastatic tumor cells, the adoptive cell therapy with T or NK cells eradicates established tumors and the T and NK cells are resistant to PD-1/PD-L1, CTLA-4/B7, and TGF-β mediated immune suppression.

A murine melanoma tumor model was used to ensure that the results seen in Cbl-b silenced human PBMCs in vitro are likely to be translatable into cancer immunotherapy in patients. T-cells were prepared from normal mice and silenced using a procedure similar as described in WO 2009/073905. The transfected T-cells were adoptively transferred along with ovalbumin-loaded dendritic cells into tumor bearing mice (ovalbumin-expressing B16 melanomas). Tumor inoculated mice received T/dendritic cell transfer 7 and 14 days after inoculation with tumor cells. Tumor size was measured up to a 1 month after tumor cell inoculation. The results showed that adoptive transfer of Cbl-b silenced murine CD8+ T-cells significantly decreased tumor burden (Hinterleitner R, Gruber T, Pfeifhofer-Obermair C, et al. Adoptive transfer of siRNA Cblb-silenced CD8+ T lymphocytes augments tumor vaccine efficacy in a B16 melanoma model. PLoS One. 2012;7(9):e44295. doi:10.1371/journal.pone.0044295).

T-cells isolated from tumor-draining lymph nodes of mice that had received transferred Cbl-b silenced T-cells were isolated 5 days after the adoptive cell transfer event and rechallenged with tumor-cell loaded dendritic cells. Sustained T-cell activity was demonstrated in terms of continued ability to produce both IFN-γ and IL-2.

There were no overt signs of autoimmunity in mice administered Cbl-b siRNA transfected CD8+ T-cells across a range of tissues.

In mice bearing B16-SIY melanoma lesions, three administrations of 5 × 10⁶ Cbl-b siRNA transfected T-cells resulted in a significant reduction in tumor volume compared with administration of control siRNA transfected T-cells. In a similar study with B16-SIY melanoma, mice were administered Cbl-b siRNA T-cells twice (on Days 9 and 14) and on Day 21 lymph nodes were taken and the characteristics of immune cells were analysed. For CD4+ T-cells, CD8+ T-cells and NK cells isolated from lymph nodes, expression of programmed cell death 1 (PD-1) was significantly lower than seen with untreated and animals receiving control siRNA transfected T-cells. As PD-1 is involved in down-regulating the immune system, this would suggest that Cbl-b silencing is also able to disrupt mechanisms usually involved in suppressing immune cell inflammatory activity at the tumor site.

In a murine hepatocellular cancer model, mice were inoculated with Hepa1-6 cells and then treated 7 days later with 5×10⁶ T-cells that had previously been transfected with Cbl-b siRNA. Administration of Cbl-b transfected T-cells reduced tumor volume by 87% by Day 14, compared with animals given control-transfected T-cells; inhibition of tumor growth was apparent for the duration of the 23-day study.

In a murine colon carcinoma model, mice were inoculated with MC38 cells and then treated 7 days later with 5×10⁶ T-cells that had previously been transfected with Cbl-b siRNA. Administration of Cbl-b transfected T-cells reduced tumor volume by 41% by Day 14, compared with animals given control-transfected T-cells; inhibition of tumor growth was apparent for the duration of the 20-day study.

Hence in the murine model reduced levels of PD-1 expressing immune cells in mice receiving Cbl-b silenced T cells and anti-tumor activity of T cells silenced for Cbl-b against MC38 colon carcinoma can be observed.

Tumor growth is inhibited in Cbl-b knock out mice as well as tumor volume is reduced following adoptive cell therapy targeting Cbl-b (Loeser, S. et al. (2007). Spontaneous tumor rejection by Cbl-b-deficient CD8+ T cells. JEM (204), S. 879-891) .

### Specification of Drug Product resulting from the method of invention

### Identity and Viability

To qualify the closed manufacturing process, drug products of 12 runs were tested for identity and viability (percentage 7-AAD negative cells of CD3+, CD14+, CD16+, CD19+ cells) and purity (percentage CD3+, CD14+, CD16+, CD19+ cells) of all living cells by flow cytometry. In addition, impurities were measured as percentage CD235+ RBCs, CD41+ PLTs and residual CD3/CD14/CD16/CD19 negative cells within CD45 positive leukocytes considered as putative granulocytes. Mean value ± SD is shown. WBC: white blood cells, RBCs: red blood cells, PLTs: platelets.

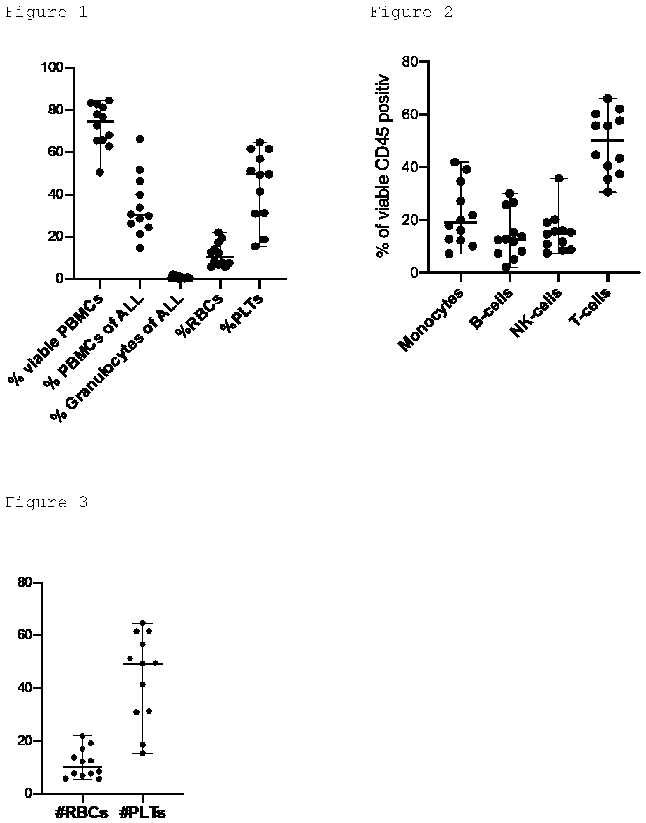

In all experiments the starting material was composed of a mean of approximately 73% viable leukocytes. Figure 1 and Table 1 depict sfFACS data demonstrating composition of drug product.

**Table 1. sfFACS data demonstrating composition of drug product**

| | % viable WBC | % WBC of ALL | % Granulocytes of ALL | %RBCs | %PLTs |
|---|---|---|---|---|---|
| Number of runs | 12 | 12 | 12 | 12 | 12 |
| Minimum | 50.7 | 14.8 | 0.310 | 5.77 | 15.6 |
| Maximum | 84.5 | 66.3 | 2.26 | 22.1 | 64.8 |
| Range | 33.9 | 51.6 | 1.95 | 16.4 | 49.2 |
| Mean | 72.8 | 34.6 | 0.931 | 11.7 | 44.5 |
| Std. Deviation | 10.4 | 14.4 | 0.577 | 5.54 | 16.8 |
| Std. Error of Mean | 2.99 | 4.16 | 0.167 | 1.60 | 4.85 |
| | | | | | |
| Lower 95% CI of mean | 66.2 | 25.4 | 0.564 | 8.22 | 33.8 |
| Upper 95% CI of mean | 79.4 | 43.7 | 1.30 | 15.3 | 55.1 |

### Characterization

By using sfFACS the drug product was analyzed according to their cell composition concerning effector cells (Percentage of CD45 positive cells). About 50% of cells were T-cells, whereas monocytes, B-cells and NK-cells were approximately below 20%. Figure 2 and Table 2 depict the sfFACS data illustrating characterization of drug product.

**Table 2. sfFACS data illustrating characterization of drug product**

| | Monocytes | B-cells | NK-cells | T-cells |
|---|---|---|---|---|
| Number of values | 12 | 12 | 12 | 12 |
| Minimum | 7.11 | 2.02 | 7.29 | 30.6 |
| Maximum | 41.8 | 30.2 | 35.7 | 66.1 |
| Range | 34.7 | 28.2 | 28.4 | 35.5 |
| Mean | 21.7 | 14.2 | 15.3 | 49.1 |
| Std. Deviation | 11.6 | 8.93 | 7.64 | 11.8 |
| Std. Error of Mean | 3.35 | 2.58 | 2.21 | 3.42 |
| Lower 95% CI of mean | 14.4 | 8.56 | 10.4 | 41.6 |
| Upper 95% CI of mean | 29.1 | 19.9 | 20.1 | 56.6 |

### Characterization of impurities

Furthermore, all cells were characterized to define the number of impurities, like erythrocytes and platelets. High amounts of platelets (44.5%) and about 11.7% of erythrocytes were detectable. Figure 3 and Table 3 depict sfFACS data illustrating characterization of impurities of drug product.

**Table 3. sfFACS data illustrating characterization of impurities of drug product.**

| | #RBCs | #PLTs |
|---|---|---|
| Number of values | 12 | 12 |
| Minimum | 5.77 | 15.6 |
| Maximum | 22.1 | 64.8 |
| Range | 16.4 | 49.2 |
| Mean | 11.7 | 44.5 |
| Std. Deviation | 5.54 | 16.8 |
| Std. Error of Mean | 1.60 | 4.85 |
| Lower 95% CI of mean | 8.22 | 33.8 |
| Upper 95% CI of mean | 15.3 | 55.1 |

### Example 1: Manufacturing of Cbl-b silenced PBMCs

An autologous cell therapy using Cbl-b checkpoint inhibitor to treat various cancers is described. Example 1 describes an autologous cell therapy targeting Cbl-b with siRNA based on highly specific activation of tumor reactive blood cells.

This example evaluates the effects of administering autologous cellular immunotherapy derived from a patient's own PBMCs that are transiently transfected with a Cbl-b siRNA molecule ex-vivo, whereas Cbl-b is silenced to reduce Cbl-b protein levels, to patients with advanced solid tumors. The transfected PBMCs are then administered back to the patient. The transfected and purified PBMCs are also referred to as produced Drug Product.

Immune cells are sampled, e.g., by leukapheresis, Cbl-b is silenced in the immune cells and the activated cells can be re-infused into the patient. The Drug Product is an autologous cellular immunotherapy derived from a patient's own peripheral blood mononuclear cells (PBMCs) that are transiently transfected with a Cbl-b specific small interfering RNA (siRNA) molecule that is able to reduce Cbl-b protein levels.

The described cell processing system efficiently transfers Cbl-b specific siRNA into purified PBMCs in a closed system. Cbl-b siRNA transfected PBMCs are administered back to the patient.

Cbl-b is a key negative regulator of antitumor immunity. It limits the reactivity of most types of immune cells, particularly lymphocytes and NK cells. A range of nonclinical data demonstrate that Cbl-b inhibition simultaneously overrides multiple relevant immune "checkpoints", and modulates sensitivity to regulatory T-cells, suppression by transforming growth factor β, and immune regulation by both cytotoxic T-lymphocyte-associated protein (CTLA)-4 and programmed cell death ligand 1 (PD-L1)/PD-1 pathways. Adoptive cellular immunotherapy with Cbl-b silenced murine T-cells significantly inhibited tumor growth in syngeneic mouse models. The Cbl-b silenced murine T-cells were synergistic with anti-PD1, supporting the potential utility of Cbl-b silenced human T-cells in combination with immune checkpoint inhibitors (CPIs) (Tang et al, 2019).

Drug product comprises autologous human PBMCs transfected ex vivo with siRNA that silences Cbl-b messenger ribonucleic acid. The Cbl-b silenced PBMCs are reinfused back to the patient, where they can respond to tumor antigen stimulation with enhanced proliferation and cytokine production. Cbl-b silenced cytotoxic T-cells and NK cells can directly mediate tumor cell killing. Cbl-b silenced B and mononuclear cells can serve as antigen-presenting cells and elicit more vigorous immune responses against tumor antigens.

PBMCs are transfected with candidate siRNA by electroporation and the mRNA is taken up into the immune cells. The inhibition of Cbl-b triggers immune reaction upon cell activation. The strong correlation of Cbl-b silencing in humans of both on mRNA and protein level to enhance immune response can be read out by cytokine secretion of IL-2, IFN-γ, and TGF-β and TCR stimulation.

High transfection rates of >95% into various immune cells relevant for anti tumor activity are achieved, whereby the Cbl-b silencing efficiency in immune cells is >90%.

Cbl-b silenced primary human T cells isolated from human PBMCs of healthy donors show a higher expression of activation marker and an increased secretion of IL-2 and IFN-γ upon T cell receptor stimulation in vitro.

Cbl-b silenced human NK cells respond stronger to tumor cell contact, enhance NK cell activation by tumor cell contact, secrete higher levels of cytotoxic granzyme B, and kill tumor cells more efficiently.

In pre-clinical studies Cbl-b deficient immune cells have led to the shrinkage and complete disappearance of tumor grafts in mice, durable antitumor memory, and growth inhibitory effects on non-injected tumors. Inactivation of Cbl-b in PBMCs and returning the cells to a patient is considered to induce a de novo antitumor immune response, leading to improved priming and activation of tumor-specific cytotoxic CD8+ T cells and increased CD8+ T cell infiltration into the tumor. Thus, silencing cells with Cbl-b siRNA and using transfected cells in a therapy may be effective to treat tumors which are primarily non-responsive (refractory) to anti-PD-1/L1 checkpoint inhibitors as well as tumors with acquired resistance to checkpoint inhibitors.

### Description of Manufacturing Process and Process Controls

For the cellular therapy, each batch is defined as the amount of freshly manufactured, not cryopreserved, autologous PBMCs containing Cbl-b silencing siRNA, as required for a single treatment cycle per patient. PBMCs are obtained from a single standard leukapheresis procedure of the respective patient, resulting in a target cell number of not less than 0.9 × 10⁸ PBMCs per kg body weight contained in approximately 200 mL. PBMC processing is carried out in a fully closed system using an appropriate amount of the leukapheresis product of not less than 0.9 × 10⁸ PBMCs per kg body weight.

Preferably the leukapheresis and the manufacturing of transfected cells as Drug Product are performed at the same site. Therefore, no transport between the collection site and the manufacturing site is required.

Transfected cells are manufactured employing a fully closed, aseptically inter-connected, 3-step manufacturing process (MAP), which uses the LOVO PBMC refinement device (Fresenius Kabi AG, Bad Homburg, Germany) and the MaxCyte ExPERT^{™} GTx Transfection System (MaxCyte, Inc. Gaithersburg, MD, USA).

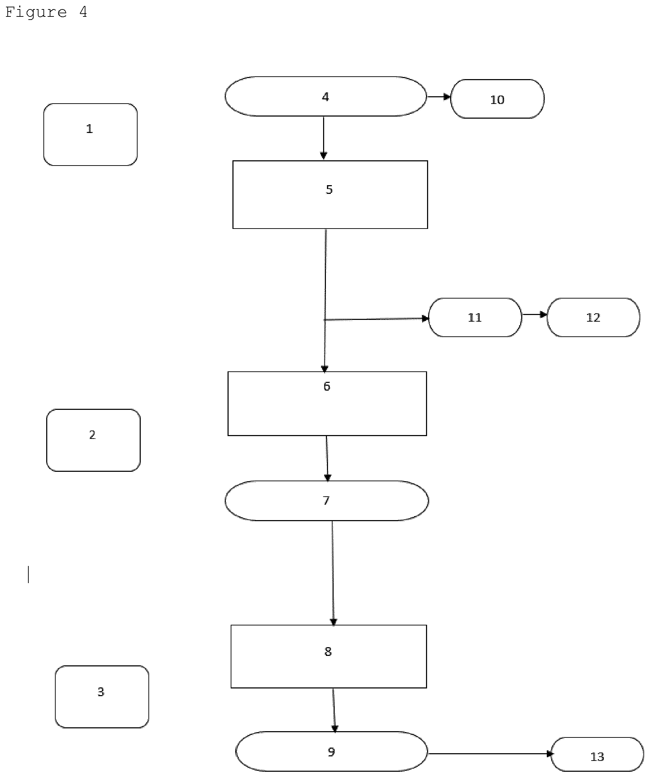

A flow chart of fully closed manufacturing process comprising step I, step II, and step III for the generation of Drug Product is shown in Figure 4.

The chart comprises the manufacturing steps I 1, step II 2, and step III 3.

Before the manufacturing process starts samples are collected and prepared, preferably unstimulated immune cells from biological liquid and/or tumor resected from the patient, in step 4, that is performed by either leukapheresis or processing of tumor samples. When performing a tumor resection, the tumor sample is processed to singular cells and will be further processed.

In step 5 the sample, especially the immune cells, is purified and resuspended and afterwards the transfection, preferably via electroporation, of the sample, especially the purified immune cells, is performed in step 6.

The transfected cells are obtained in step 7 and may be resuspended in electroporation buffer after final collection in a container.

In the next step 8, the transfected immune cells are transferred to a container, preferably an infusion bag, and the immune cells are rebuffered to the final formulation and finally the Drug Product is obtained in a container for infusion in the last step 9.

Controls 10, 11, 12, and 13 are performed and they are taken from step 4, control IPC01 10, after step 5, controls IPC02 11 and S01 12 are performed from step 9, control S02 13.

The manufacturing process (MAP) for the generation of transfected cells comprises MAP step I 1, i.e., the PBMC purification and resuspension, followed by MAP step II 2, i.e., the PBMC electroporation.

PBMCs from a patient's leukapheresis are the starting material as shown in step 4 of the flow chart (Fig. 4). A sample, control IPC01 10, is aseptically drawn before the beginning of manufacturing process from the starting material, i.e. leukapheresis product, to measure PBMC number and content, preferably WBC quantity and haematocrit by complete blood count (CBC) as well as to test for sterility as shown in step 10 of the flow chart. CBC parameters are required for programming the LOVO device of MAP step I 1.

In MAP step I the PBMC purification and resuspension is performed by adding PBS/EDTA solution, an ammonium chloride potassium lysis buffer and an electroporation buffer, as shown in step 5 of the flow chart.

MAP step II 2 comprises the transfection via electroporation with Cbl-b siRNA, what is represented in step 6 of the flow chart. With a duration of 20 +/- 5 minutes approximately 1×10¹⁰ purified PBMCs can be efficiently silenced for Cbl-b expression showing a comparable silencing efficiency on mRNA level as compared to Amaxa Nucleofector 4D. By the means of MaxCyte ExPERT^{™} GTx electroporation an average silencing of 65% on mRNA level leads to a reduction of Cbl-b protein below the detection limit of mass spectrometry (MS)-based Cbl-b detection. Furthermore, effective silencing correlates with robust and consistent induction of a mean, 8-fold IL-2 release in comparison to non-silenced no-pulse control 12.

MAP step I 1 and step II 2 are aseptically inter-connected without intermediate PBMC reprocessing. Used buffers and siRNA are aseptically introduced into the manufacturing process.

In the process of cell transfection, control sample IPC02, represented as step 11 of the flow chart, which comprises the specification control sample S01 before electroporation of MAP step II 2, illustrated as step 12 of the flow chart, is aseptically drawn from the cell bag containing purified PBMCs. IPC02 also serves as the no pulse control (NPC) to measure the immune default status of PBMC activity (Cbl-b expression) and potency (IL-2 secretion) before siRNA transfection is performed by PBMC electroporation in MAP step II 2 as S01 12.

Both, Cbl-b and IL-2 levels of no pulse control 12 provide information to specify activity and potency in the final Drug Product.

Hence, the manufacturing process enables automated, closed PBMC purification step, as described in MAP step I 1, followed by an automatically performed, closed PBMC electroporation step, as described in MAP step II 2 to transfect purified autologous PBMCs from apheresis products with Cbl-b specific siRNA.

Control IPC 01 10 of the starting material is used as input control to determine the white blood cell and platelet quantity as well as the haematocrit. Control IPC02 11 is drawn as no pulse control (NPC) and only serves as the specification control S01 12 for final Drug Product release. Control S01 12 defines the patient immune status before Cbl-b silencing and is therefore used to normalize activity and potency measurements on Drug Product.

Purified leukopak PBMCs are electroporated to introduce Cbl-b siRNA, potentially leading to cell damage, hence reduced cell viability and cell aggregation. Consequently, a substantial fraction of cells after transfection 6 consists of non-viable and aggregated cells as well as electroporated granulocytes, erythrocytes, and platelets, which are to a strong degree depleted during the last MAP step III, comprising, and illustrated as step 8 of the flow chart, wherein the transfected immune cells are rebuffered to the final formulation and Drug Product is received in the final container, as illustrated in step 9 of the flow chart.

Cell aggregation by macroscopic visual inspection (appearance) and PBMC viability by flow cytometry (identity) cannot be tested in the transfection product, therefore both parameters are specified only for the transfected and purified Drug Product, illustrated in step 9 and 13 of the flow chart, respectively. Impurities, such as granulocytes or non-nucleated cells, like PLTs and RBCs, are specified on the Drug Product, shown in step 9 of the flow chart, by flow cytometry evaluated as PBMC negative cells of all blood cells, that functions as indirect purity measurement. Excipient buffers and siRNA are strongly depleted in the final Drug Product formulation.

Both cell processing systems utilize single-use-tubing sets for each patient batch, which are inter-connected by aseptic welding of the out-put PBMC bag from the LOVO tubing set to the ExPERT^{™} GTx tubing set without additional PBMC reprocessing. PBMC and buffer bags as well as sterile sample withdrawal-ports for control of the starting material/leukapheresis product and Controls IPC01 10 and IPC 02 11 are aseptically connected using the Terumo sterile tubing welder (TerumoBCT, Belgium).

All manipulation steps are performed by use of e-beam sterilized single-use equipment.

The manufacturing process requires bag filling of RBC lysis buffer and electroporation buffer, which is carried out under aseptic conditions. When employing the current manufacturing process set-up, Cbl-b siRNA is injected into the LOVO of MAP step I 1 out-put PBMC bag via a sterile filter connected to the Control IPC01 10 withdrawal-port via a swabable Luer port in a laminar air flow cabinet before proceeding with the electroporation process. The laminar air flow cabinet is regularly monitored by settle plates according to GMP standards.

MAP step II 2 of Drug Substance manufacturing and MAP step III 3 are also aseptically inter-connected without intermediate immune cell, preferably PBMC, reprocessing. Drug product control S02 13 is aseptically drawn from Drug Product to measure the Quality Control parameters, i.e., appearance, identity, quantity, purity, safety, activity, and potency.

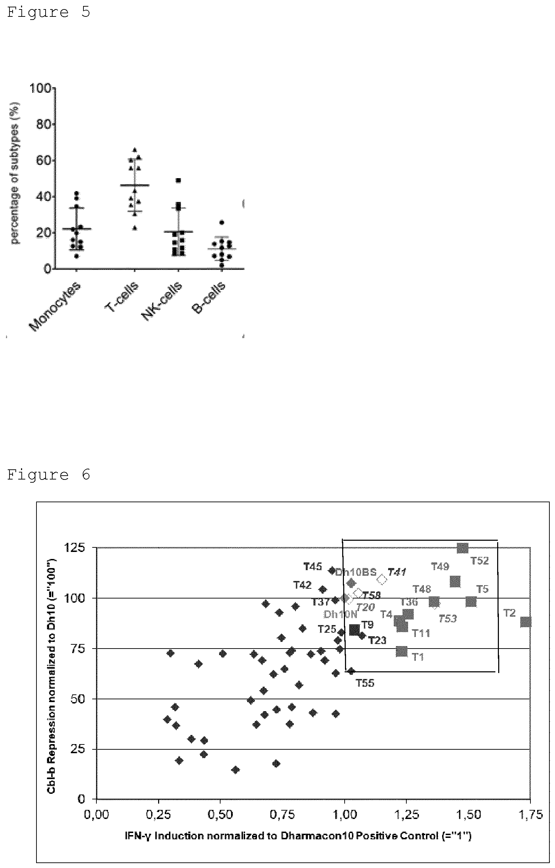

In Figure 5 the immune subtype distribution in Cbl-b siRNA electroporated PBMCs using the LOVO and MaxCyte electroporation device is shown, and the distribution of immune cell sub types measured by lineage marker expression of CD14 (monocytes), CD3 (T cells), CD16 (NK cells), and CD19 (B cells) by flow cytometry in apheresis products processed with Manufacturing process is illustrated. Processed PBMCs underwent a final purification and rebuffering step as it has been established for final Drug Product formulation (n=11 healthy donors).

### Starting Material (Leukopak) for Drug Substance Manufacturing

Patient's PBMCs are collected by leukapheresis according to standard procedures (e.g., Terumo Optia apheresis device) to enable enrichment of lymphocytes and monocytes, while granulocytes, thrombocytes and erythrocytes are depleted. The leukapheresis procedure should yield not less than 0.9 × 10⁸ PBMCs per kg body weight (target: 1.2 + 0.3 × 10⁸ PBMCs per kg body weight), which is measured by complete blood count (CBC) as in-put control 10 to furthermore specify the leukopaks (PBMC content is <70(% PBMCs of total WBCs), Haematocrit is ≤3.5% and platelet count is <2.0×10⁶ PCT/µL). Prior to the collection, the following serological viral safety tests are performed for patient safety screening: HIV I/II, HBV, HCV and Syphilis). PBMCs are collected using ACD-A buffer comprising Citric Acid, Dextrose Monohydrate, Sodium Citrate Dihydrate, and Water, which is diluted 1:12 during leukapheresis procedure resulting in the final concentration of ACD-A components in leukopaks.

The finished patient's leukapheresis product is stored at 5±3°C without shaking for ≤ 24 hours.

### MAP Step I - LOVO - PBMC Purification & Rebuffering:

The invention allows automated red blood cell lysis and platelet depletion followed by rebuffering of PBMCs into electroporation buffer for the following MAP step II. Within this closed tubing set PBMCs are purified and resuspended in electroporation buffer (MaxCyte, USA), while introduced components of apheresis buffer, PBS/EDTA wash buffer (CliniMACS buffer, Miltenyi, Germany) and ammonium chloride potassium lysing buffer (ACK) (Thermo Scientific, Massachusetts) are efficiently depleted.

The LOVO system allows standardized isolation of PBMCs to a similar quantity (p > 0.05) as Ficoll separation, which is highly operator dependent.

Before starting with PBMC purification of MAP step I 1, buffer bags and a PBMC collection bag are aseptically connected to the respective port at the single-use LOVO tubing set, which subsequently is mounted onto to the LOVO device.

Before starting the LOVO programm a cell strainer to filter cell aggregates upstream of the final container closure system bag needs to be inserted in the correct orientation to the single-use tubing set. The welds must be opened correctly to ensure free flow of the Drug Product to the final enclosure. After intake of leukopak PBMCs into the Lovo tubing set, the leukopak container bag is flushed with PBS/EDTA. At this step the bag may be agitated to minimize cell loss.

To program the LOVO device, the leukopak starting material needs to be specified by testing for white blood cell count (WBC) and hematocrit (HCT) count. The leukopak starting material is verified to contain 0.9 - 1.5 × 10⁸ PBMC/kg body weight. In case of the PBMC content exceeding 1.5 × 10⁸ PBMC/kg body weight, assuming a theoretical maximum body weight of 100 kg, the maximum processable PBMC number of MAP Step I 1 (1.5 × 10¹⁰ PBMCs) would be exceeded. Therefore, in such case, the calculated excess volume is removed from the leukopak bag before connection to the LOVO device.

After entering the required LOVO program parameters and completion of the LOVO set-up and priming procedure, the leukopak bag is aseptically connected to the input port at the LOVO tubing system. The entire leukopak content is then automatically transferred into the tubing system to run MAP step I 1. A successful completion of the LOVO run recorded by internal device monitoring system is reported for batch release.

In the detailed process of MAP step I, six wash cycles deplete smaller-sized cells and cell debris from 200 mL leukopak starting material by spinning membrane filtration, which enables PLT depletion, while RBCs are removed by an incubation cycle using an RBC lysing agent, the ammonium chloride potassium lysing buffer lysis buffer. After the addition of the ACK lysing buffer, the operator is prompted to mix the incubation bag without delay. Only after confirmation, the 10-minute countdown of the incubation commences. Any delay can increase incubation time and possibly result in cell loss. Following the RBC lysis wash cycle, PBMCs are washed using PBS/EDTA buffer and rebuffered into the MaxCyte electroporation buffer. The entire MAP step I procedure takes ≤ 1 hour to collect ≤ 55 mL PBMCs resuspended in electroporation buffer.

At the end of the sample purification and resuspension procedure, PBMCs resuspended in electroporation buffer are pumped into the PBMC collection bag, which is then aseptically reconnected to the ExPERT^{™} GTx tubing set to directly continue with MAP step II (MaxCyte ExPERT^{™} GTx - PBMC Transfection via Electroporation). At this point, the processing assembly with the PBMC bag attached is stored in a refrigerator at 2-8°C for 10 to 15 minutes for pre-cooling until proceeding with MAP step II.

### MAP Step II - MaxCyte ExPERT^{™} GTx - PBMC Transfection via Electroporation

Using the ExPERT^{™} GTx electroporation system high PBMC numbers can be processed automatically by consecutive electroporation in a closed system. Before starting the electroporation procedure, in-process control IPC02 (specification control sample S01) is drawn aseptically via the swabable Luer port from the PBMC bag in a laminar flow hood to collect a no pulse control (NPC) before electroporation. Control sample S01 is stored for Drug Product (DP) specification testing at room temperature until completion of Drug Product manufacturing and drawing of Drug Product sample S02.

After in-process control IPC01 withdrawal, the entire content of one vial of ready-to-use Cbl-b siRNA is injected into the PBMC bag with a syringe through a 0.2µm syringe filter connected to the swabable Luer in-process control IPC01 withdrawal port thereby assuring a closed MAP.

After siRNA injection, the ExPERT^{™} GTx tubing set connected to the PBMC collection bag is mounted onto the MaxCyte ExPERT^{™} GTx Transfection System to run closed automated electroporation.

siRNA transfection via electroporation is initiated using the MaxCyte ExPERT^{™} GTx Transfection System electroporation software by starting the designated electroporation routine ("expanded T cell 3 pro"). Electroporation parameters are according to manufacturer's instructions.

A maximum of 19 consecutive electroporations are performed automatically to process a maximum of 55 mL PBMC suspension in a time frame of 20 +/- 5 minutes which enables the manufacture of one batch of ≤ 56mL suspended transfected cells.

Autologous PBMCs transfected with Cbl-b siRNA, is resuspended in electroporation buffer after final collection in a PBMC bag, which is a component of the ExPERT^{™} GTx tubing set.

### siRNA

The human Cbl-b gene is defined by the UniGene entry in GenBank Hs.430589. The mRNA sequence of Cbl-b is defined by GenBank entry NM_170662.3, single-nucleotide polymorphism information was retrieved from GenBank and all relevant polymorphisms were confirmed by sequencing.

The siRNA allows sustained Cbl-b silencing (not less than 4 days) and cytokine induction (esp. IFN-γ expression).

Figure 6 illustrates optimized siRNA, when PBMCs were isolated and Cbl-b was silenced. Sixty-five siRNAs were designed, synthesized, and then screened for Cbl-b silencing and induction of cytokine production in PBMCs. A selection of siRNAs, which both induced IFN-γ expression and resulted in robust Cbl-b silencing, were selected for re-synthesis. Following anti-CD3/28 stimulation, IFN-γ production (ELISA) and Cbl-b levels (icFACS) in T-cells were determined and expressed relative to a reference commercial siRNA (Dharmacon). The frame highlights siRNAs showing strong silencing efficacy in correlation with strong increase in IFN-γ production.

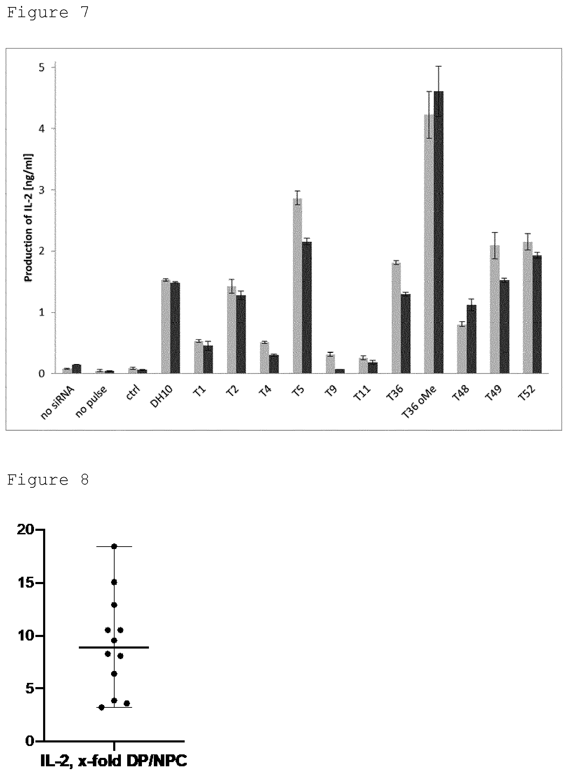

PBMCs were isolated and Cbl-b silenced followed by stimulation using anti-CD3/28 antibodies as shown in Figure 7. IL-2 production was determined after 3 days of stimulation by ELISA. Grey and black bars designate independent duplicates for transfection and stimulation. Ctrl: control electroporation using non-target siRNA.

60 siRNAs were synthesized and screened for Cbl-b silencing and enhancement of cytokine production of TCR stimulated PBMCs as depicted in Table 4.

**Table 4. List of modified nucleotide sequences**

| **SEQ ID NO:** | **strand** | **sequence (5'>3')** | **duplex number** |
|---|---|---|---|
| 1 | sense | GUGAGAAUGAGUACUUUAA11 | 1 |
| 2 | antisense | UUAAAGUACUCAUUCUCAC11 | |
| 3 | sense | 8U8AGAAUGAGUACUUU6A11 | 2 |
| 4 | antisense | UU6AAGUACUCAUUCU7A711 | |
| 5 | sense | 8U8AGAAUGAGUACUUUAA11 | 3 |
| 6 | antisense | 55AAAGUACUCAUUCUCAC11 | |
| 7 | sense | 8U8AGAAUGAGUACUUUAA11 | 4 |
| 8 | antisense | U5AAAGUACUCAUUCUCAC11 | |
| 9 | sense | 8U8AGAAUGAGUACUUU6A11 | 5 |
| 10 | antisense | U5AAAGUACUCAUUCUCAC11 | |
| 11 | sense | 8U8AGAAUGAGUACUUU6611 | 6 |
| 12 | antisense | 55AAAGUACUCAUUCUCAC11 | |
| 13 | sense | GUGAGAAUGAGUAC 5 5U 6A11 | 7 |
| 14 | antisense | U5AAAGUACUCAUUCUCAC11 | |
| 15 | sense | GUGAGAAUGAGUAC55U6611 | 8 |
| 16 | antisense | 55AAAGUACUCAUUCUCAC11 | |
| 17 | sense | G5G6G6A5G6G5A7U5U6A11 | 9 |
| 18 | antisense | UUAAAGUACUCAUUCUCAC11 | |
| 19 | sense | GUGAGAAUGAGUACUUUAA11 | 10 |
| 20 | antisense | 5U6A6G5A7U7A5U7U7A711 | |
| 21 | sense | 8U8A8AAUGAGUACUUU6A11 | 11 |
| 22 | antisense | UU6AAGUACUCAUU7U7A711 | |
| 23 | sense | GUGAGAAUGAGUACUUUAA11 | 12 |
| 24 | antisense | 22AAAG2A323A22323A311 | |
| 25 | sense | G2GAGAA2GAG2A3222AA11 | 13 |
| 26 | antisense | UUAAAGUACUCAUUCUCAC11 | |
| 27 | sense | GUG6GA6UG6GUACUUUAA11 | 14 |
| 28 | antisense | 22AAAG2A323A22323A311 | |
| 29 | sense | 8U8AGAAUGAGUACUUUAA11 | 15 |
| 30 | antisense | UUAAAGUACUCA22323A311 | |
| 31 | sense | G2GAGAA2GAGUACUUUAA11 | 16 |
| 32 | antisense | UUAAAGUACUCAUUCUCAC11 | |
| 33 | sense | 85GAGAAUGAGUACUUU6611 | 17 |
| 34 | antisense | UUAAAGUACUCAUUCUCAC11 | |
| 35 | sense | GUG6G66UG6GU6CUUU6611 | 18 |
| 36 | antisense | UUAAAGUACUCAUUCUCAC11 | |
| 37 | sense | GUG4G44UG4GU4CUUU4411 | 19 |
| 38 | antisense | UUAAAGUACUCAUUCUCAC11 | |
| 39 | sense | G5GAGAA5GAG5ACU5UAA11 | 20 |
| 40 | antisense | UUAA8GUACUCAUUC5CAC11 | |
| 41 | sense | G2G4G442GAGUACUUUAA11 | 21 |
| 42 | antisense | UUAAAGUACUCAUUCUCAC11 | |
| 43 | sense | GUGAGAAUGAGUACUUUAA11 | 22 |
| 44 | antisense | 2U4A4G24CUCAUUCUCAC11 | |
| 45 | sense | G5G6G665GAGUACUUUAA11 | 23 |
| 46 | antisense | UUAAAGUACUCAUUCUCAC11 | |
| 47 | sense | G5G6G665GAGUACUUUAA11 | 24 |
| 48 | antisense | UUAAAGUACUCA2U3U3A311 | |
| 49 | sense | G2G4G442GAGUACUUUAA11 | 25 |
| 50 | antisense | UUAAAGUACUC6U5C5C6C11 | |
| 51 | sense | 8U8A8AAU8A8UACUUUAA11 | 26 |
| 52 | antisense | UUAAAGUACUCAUUCUCAC11 | |
| 53 | sense | G5GAGAA5GAG5AC555AA11 | 27 |
| 54 | antisense | UUAAAGUACUCAUUCUCAC11 | |
| 55 | sense | GUG6G66UG6GU6CUUU6611 | 28 |
| 56 | antisense | UUAAAGUACUCAUUCUCAC11 | |
| 57 | sense | GUG6GAAUG6GU6CUUU6A11 | 29 |
| 58 | antisense | 5UAAAGUACUCAU5CUCAC11 | |
| 59 | sense | GU8A8AAU8A8UACUUUAA11 | 30 |
| 60 | antisense | UUAAAG5ACUCAUUC5CAC11 | |

| | | | |
|---|---|---|---|
| 1= dT (deoxy-thymidine) 2= 2'-F-U 3= 2'-F-C 4= 2'-F-A 5= 2'-OMe-U 6 = 2'-OMe-A 7 = 2'-OMe-C 8= 2'-OMe-G | | | |

siRNAs can differ in efficacy of silencing but also in persistence inside cells. Generally, modifications like methylated or fluorinated bases enhance stability, but an excessive degree of modifications decreases silencing efficiency. Ideally, siRNAs cause efficient and long-lasting silencing, enabling enhanced cell stimulation for a sustained period of time and also for cells which get stimulated in vivo by APCs only a few days after administration. Accordingly, an experiment was set up to monitor early (day 1) and late (day 7) levels of Cbl-b and consequential effects of Cbl-b silencing on T cell activation for several siRNA modifications as listed in Table 4.

PBMCs were isolated by Ficoll centrifugation from a healthy donor (buffy coat). For transfection, cells were electroporated by using an Amaxa Nucleofector device (pulse program Y01) with 0.2 cm gap width cuvettes. The cells were either mock-transfected or transfected with the respective Cbl-b siRNAs (6µM). The cells were rested overnight in Xvivo 15 serum-free medium. On day 1(d1), cells were harvested, counted and distributed into 4 aliquots to perform intracellular staining and flow cytometry analysis for Cbl-b levels (co-stained for CD4 and CD8), an RT-PCR for Cbl-b mRNA expression, an antiCD3/28 stimulation (overnight), followed by ELISA for cytokine production (IL-2, IFN-g and TNF-a) and analysis of cell surface expression of activation markers by flow cytometry (CD25, CD69). Remaining cells were cultured for 6 additional days in the presence of recombinant human IL-2 (rhu IL-2, 5ng/mL). On day 7 (d7), these remaining cells were harvested & and icFACS, RT-PCR & ELISA/FACS analysis was performed as for day 1 (d1). When sufficient cells were available, Western blot analysis for Cbl-b protein was performed. The Western Blot data from day 7 confirmed the icFACS data.

Most modified siRNAs can enhance IL-2 production as consequence of enhanced T cell activation by Cbl-b silencing.

Some modified siRNAs can enhance IL-2 production as consequence of enhanced T cell activation by sustained Cbl-b silencing over 7 days.

The optimal Cbl-b siRNA is unique in the human transcriptome, as can be confirmed by blast search for homologous sequences.

For manufacturing of the siRNA, a sense and antisense single strands are chemically synthesized with a commercially available OP100 synthesizer (Biospring Gesellschaft fur Biotechnologie mbH, Frankfurt, Germany). After syntheses the crude oligonucleotides are cleaved and deprotected and then purified by AEX-HPLC. After purification, the single strands are desalted, concentrated, annealed, and lyophilized.

The siRNA is processed into a ready-to-use formulation ("formulated siRNA") by Symbiosis Pharmaceutical Services (Stirling, Scotland) at a concentration of 5.0 mg per 1 mL phosphate-buffered saline, which can be used to transfect approximately 1.2×10⁸/kg PBMCs from leukopak in-put material. The siRNA undergoes a final sterile-filtration step and is then transferred under aseptic conditions into glass vials. Stoppered ('ready-to-use') glass vials are stored at -20±5°C. The frozen siRNA is thawed at room temperature at the start of the process.

After a holding time of 30 to 60 minutes at room temperature in a PBMC bag, transfected PBMCs are further processed for purification and resuspension by aseptically reconnecting the PBMC bag to the LOVO tubing set used for final formulation.

### MAP Step III - Drug Product Formulation

To assure fully closed manufacturing, a new LOVO single-use-tubing set for each patient batch is used. Before starting the process, the out-put PBMC bag from the ExPERT^{™} GTx tubing set from MAP step II (designated Drug Substance) is directly connected to LOVO tubing-set and mounted onto the LOVO device. PBMC and buffer bags as well as withdrawal-ports to draw sterile Drug Product control (S02) or aseptically remove excess PBMCs for cell dosing are aseptically connected using the Terumo sterile tubing welder (TerumoBCT, Belgium). All manipulation steps are performed by use of e-beam sterilized single-use equipment.

The transfected cells are formulated for intravenous infusion in 0.9% w/v sodium chloride solution with 0.7 g/l human serum albumin (HSA) which further depletes residual PLTs and cell debris. Furthermore, electroporation buffer as the transfected cells' matrix and excess free siRNA are strongly reduced. The compendial excipients chosen for the formulation protect transfected cells from degradation and aggregation and are used for parenteral dosage forms of this cellular product. The final cell concentration is adjusted up to 4.5×10⁷ PBMCs/kg body weight in 200 mL physiological sodium chloride solution containing 0.7% human serum albumin. The quantity of PBMCs administered per dose dictates the total infusion volume. The entire MAP step III procedure takes ≤ 12 minutes to collect ≤ 200 mL PBMCs resuspended in 0,9% physiological NaCl + HSA (0.7g/l) solution (i.e. Drug Product), which then are adjusted to the target volume containing the defined PBMC number for infusion. The Drug Product is finally stored and infused from the bag directly detached after the last manufacturing step. Sixteen mL of Drug Product control S02 are drawn aseptically for immediate release testing. Upon removal of S02 the highest possible total volume of ≤ 184 mL can be obtained, which consequently contains the maximum manufacturable dose (MMD) of PBMCs for potential infusion.

Drug Product is immediately administered after manufacturing and release testing (without cryo-preservation). The quantity of PBMCs administered per dose, according to treatment regime, preferably one dose comprises 5×10⁶ to 5×10¹⁰, preferably 5×10⁷ to 5×10⁸, transfected PBMCs/kg, defines the total volume for infusion. The majority of PBMCs consists of T and NK cells.

The target volume for infusion of individual Drug Products depends on the selected target dosage of PBMCs currently defined in the clinical study protocol by ascending dosing cohorts with a given PBMC number/kg patient weight. Consequently, the target number of PBMCs is calculated by multiplying the dose per kg with the body weight of the patient. Furthermore, the actual PBMC concentration (PBMCs/mL) in the Drug Product determined by a CBC count from control sample S02 allows calculating the target volume containing the defined PBMC number for infusion. This target volume is multiplied by a density correction factor (acc. to the EDQM Guide to the preparation, use and quality assurance of blood components, 20th edition (Pharmacopoeia, 2015)) to calculate the target Drug Product bag weight (plus empty bag weight tare). This value allows the final adjustment of the Drug Product for infusion.

Technically Drug Products are reduced to the target amount by transferring the overage into a sterile connected overage bag. The remaining PBMC suspension in the Drug Product bag is weight using a qualified scale to exactly adjust the target Drug Product weight for infusion. The reported target Drug Product weight must not deviate more than ±10% from the calculated Drug Product weight containing the exact PBMC number for dosing.

The manufacturing process from PBMC purification of patient blood (leukopak) to the of the final formulation of Cbl-b silenced PBMCs (Drug Product) via cell transfection is semi-automated and carried out without isolation of intermediates. No cell hold time or additional storage of the transfected cells apply in order to shorten the manufacturing process time and maximize the number of viable PBMCs re-administered to the patient, resulting in robust PBMC viability and the maximum manufacturable dose, which is considered as the critical cell dosage for effective treatment.

Drug Product efficacy primarily relies on the occurrence of T and NK cells after final formulation of Drug Substance for infusion. Starting with apheresis over processing of apheresis products the cell disruption shows a mean value of 43% T cells and 20 % NK cells in the final Drug Product.

Drug Product characteristics are measured by release testing prior to administration and further testing, which can be completed only after infusion. Drug Product release tests before patient infusion comprises appearance (visual inspection), identity (PBMCs within nucleated cells), quantity (to be infused Drug Product containing the target PBMC number), viability (viable PBMCs), purity (PBMCs of all blood cells) and safety (endotoxin content)). The release testing prior to administration is performed by the manufacturer. Further Drug Product testing completes characterization post infusion by further determining sterility, activity (Cbl-b expression in activated Drug Product) and potency (IL-2 secretion from activated Drug Product).

Adjusted Drug Products are administered within 6 hours post manufacturing without further cell manipulation from the same cell bag.

The mRNA silencing results can be confirmed on protein level by showing a mean silencing of 100% by immunoblotting using Cbl-b specific antibodies. This correlates with a mean reduction of 4.06 ng Cbl-b/100 µg total protein in S01 to 0.89 ng Cbl-b/100 µg total protein in S02 measured by mass spectroscopic analysis of trypsin digested total protein of PBMCs.

Both devices, the LOVO PBMC refinement device and the MaxCyte ExPERT^{™} GTx Transfection System are programmed by the respective equipment manufacturer or during method development.

### Specification of a drug product resulting from siRNA-mediated silencing of cbl-b using the method of invention

The drug product (DP) is a suspension of viable leukocytes from an individual patient that has been transfected with a small interfering ribonucleic acid (siRNA) in order to reduce Cbl-b expression. Transfected autologous leukocytes are infused back into the patient. Each batch is defined as a single treatment cycle per patient using freshly manufactured (not cryopreserved) DP.

Cbl-b is considered as a negative regulator of innate and adaptive immunity, thus, being involved in tumor triggered immune evasion. As a consequence, blocking Cbl-b function upon silencing the Cbl-b mRNA in leukocyte may lead to the reactivation of immune processes that help eradicating tumor cells. Along these lines, administration of autologous, Cbl-b silenced leukocytes represents a novel approach with the concept of enhancing anti-tumor immune responses leading to a novel cancer therapy.

### Potency

### Interleukin 2 Enzyme Linked Immunosorbent Assay (ELISA)

Drug Product samples and no pulse control samples (NPC) of 12 MAP runs (n = 12 healthy individuals) were analyzed for IL-2 secretion by ELISA using supernatants from anti-CD3/28 stimulation cultures. Fold-induction of IL-2 in DPs over NPCs as reported for DP specification showing max and min induction. IL-2 fold increase comparing drug product (DP) to no pulse control (NPC) measured by ELISA are shown in Figure 8 and Table 5 with Mean values ± SEM.

**Table 5. IL-2 fold increase comparing drug product (DP) to no pulse control (NPC) measured by ELISA.**

| | IL-2, x-fold DP/NPC |
|---|---|
| Number of values | 12 |
| Minimum | 3.25 |
| Maximum | 18.4 |
| Range | 15.2 |
| Mean | 9.20 |
| Std. Deviation | 4.68 |
| Std. Error of Mean | 1.35 |
| Lower 95% CI of mean | 6.22 |
| Upper 95% CI of mean | 12.2 |

### Activity

Silencing efficiency was evaluated by measuring drug product Cbl-b mRNA expression via qPCR and correspondingly Cbl-b protein availability via Western blot analysis. Of note, stimulation drives Cbl-b expression which facilitates detection of Cbl-b, therefore cells were stimulated with anti-CD3/CD28.

### RNA Isolation and qPCR

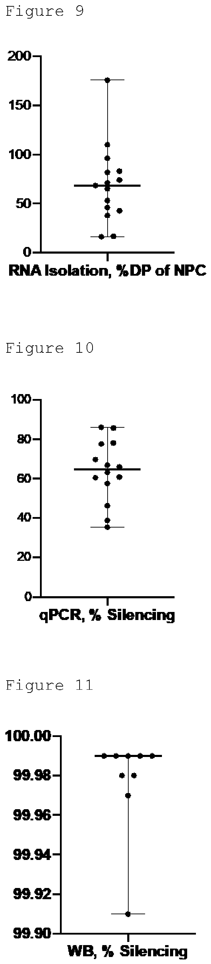

Gene silencing with siRNA causes a specific degradation of sequence complementary messenger RNA, resulting in cumulative depletion of the appropriate protein. Therefore, measuring a specific mRNA by using quantitative real time PCR (qPCR) is a suitable method to quantify the silencing efficacy of the siRNA. In these experiments a mean silencing efficiency of 63.9% on mRNA level was detectable, illustrated in Figure 9 and Figure 10 and Table 6 and 7. Figure 9 depicts Percentage of Cbl-b RNA expression of drug product (DP) in relation to no pulse control (NPC) and Figure 10 depicts Percentage of Cbl-b Silencing comparing DP and NPC measured with qPCR.

**Table 6. Relation of Cbl-b RNA expression of drug product (DP) and no pulse control (NPC)**

| | RNA Isolation, %DP of NPC |
|---|---|
| Number of values | 15 |
| Minimum | 16.3 |
| Maximum | 176 |
| Range | 159 |
| Mean | 69.2 |
| Std. Deviation | 39.8 |
| Std. Error of Mean | 10.3 |
| Lower 95% CI of mean | 47.2 |
| Upper 95% CI of mean | 91.3 |

**Table 7. Percentage of Cbl-b Silencing comparing DP and NPC measured with qPCR**

| | qPCR, % Silencing |
|---|---|
| Number of runs | 14 |
| Minimum | 35.4 |
| Maximum | 86.3 |
| Range | 50.9 |
| Mean | 63.9 |
| Std. Deviation | 15.8 |
| Std. Error of Mean | 4.21 |
| Lower 95% CI of mean | 54.8 |
| Upper 95% CI of mean | 73.0 |

### Western Blot

As described above, Cbl-b protein detection via Western blot analysis is a key analysis method to evaluate activity of the transfected cells manufacturing procedure. Of note, stimulation drives Cbl-b expression which facilitates detection of Cbl-b, therefore cells were stimulated with anti-CD3/CD28.

Nearly up to 100% efficiency in Cbl-b silencing was detected on protein level following anti-CD3/CD28 stimulation illustrated in Figure 11 and Table 8.

**Table 8. Percentage of Cbl-b silencing on protein level comparing DP and NPC using WB analysis.**

| | WB, % Silencing |
|---|---|
| Number of values | 9 |
| Minimum | 99.9 |
| Maximum | 100 |
| Range | 0.0800 |
| Mean | 100 |
| Std. Deviation | 0.0260 |
| Std. Error of Mean | 0.00866 |
| Lower 95% CI of mean | 100 |
| Upper 95% CI of mean | 100 |

### LC-MS/MS based Cbl-b quantification

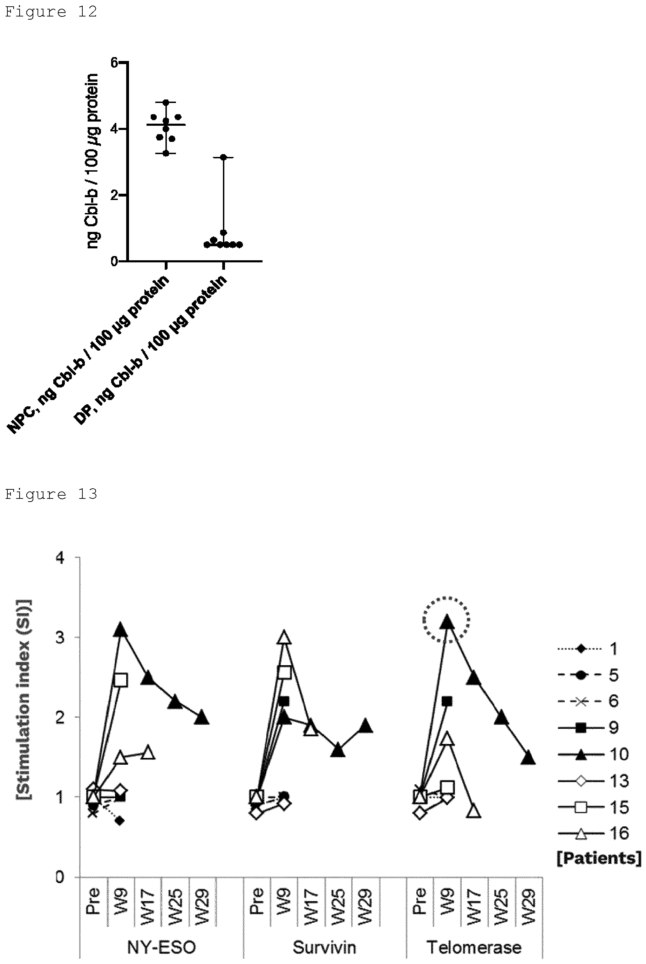

Detection of low molecular amounts as endogenous levels of Cbl-b in complex matrices, such as cell lysates needs a specialized analytical procedure to overcome those limitations. Quantification of Cbl-b protein at nanomolecular ranges were done by mass spectrometry using a sensitive and selective protein quantification assay of Cbl-b and consecutive LC-MS/MS based quantification of one selected tryptic peptide for the targeted quantification approach. Significant silencing efficacy was seen in those cell lysates comparing NPC and DP, demonstrating effectiveness of the method of the invention illustrated in Figure 12 and Table 9.

**Table 9. Cbl-b quantification in cell lysates of DP and NPC using LC-MS/MS**

| | NPC, ng Cbl-b / 100 µg protein | DP, ng Cbl-b / 100 µg protein |
|---|---|---|
| Number of runs | 8 | 8 |
| Minimum | 3.26 | 0.500 |
| Maximum | 4.80 | 3.14 |
| Range | 1.54 | 2.64 |
| Mean | 4.06 | 0.894 |
| Std. Deviation | 0.482 | 0.917 |
| Std. Error of Mean | 0.171 | 0.324 |
| Lower 95% CI of mean | 3.66 | 0.127 |
| Upper 95% CI of mean | 4.46 | 1.66 |

Results show that Drug Product induces long lasting tumor-specific T cell immunity as shown in Figure 13, where IFN-γ production in response to stimulation with peptides corresponding to NY-ESO, survivin, and telomerase of PBMC collected from selected patients, pre-treatment and at weeks (W) 9, 17, 15, and 29 are shown. Data are presented as stimulation index (SI).

The present invention shows the effect of administering autologous peripheral blood mononuclear cells in which E3 ubiquitin-protein ligase Casitas-B-lineage lymphoma protein-b has been silenced ex vivo to patients with advanced solid tumors where well-established standard of care medication has ceased to be effective.

To investigate the Drug Product's (cbl-b silenced PBMCs) effects in humans, it was assessed by two Phase 1 clinical studies in cancer patients. The open-label Phase I studies were primarily designed to evaluate safety and tolerability of transfected PBMCs in patients with inoperable, recurrent or metastatic malignant solid tumours, deemed incurable, who failed to respond to stand therapy or for whom no standard therapy was available. In addition, the studies were to determine whether there was sufficient evidence of immunologic activity to proceed with clinical development.

The first Phase I study (CCCWFU 99114) was a single-dose study to characterize the toxicity profile and establish a maximum tolerated dose (MTD). Three patients received one dose of 5 × 10⁵ transfected PBMCs/kg, three patients received one dose of 1 × 10⁶ transfected PBMCs/kg and 10 patients received one dose of 5 × 10⁶ transfected PBMCs/kg. Patients suffered from colorectal, renal cell, and pancreatic cancer. Transfected PBMCs were generally well tolerated, with mild to moderate chills developing as most patients completed the infusion. Dose-limiting toxicity was not observed. There was no immediate hypersensitivity and no evidence of autoimmune adverse effects. Therefore, this study established the feasibility and safety of administering Drug Product.

The second Phase I study (CCCWFU 03716) was a multiple-dose study to characterize the toxicity profile. The highest dose used in the first study (5×10⁶ transfected PBMCs/kg) was selected as the dose to be administered. Patients were to receive three intravenous PBMC infusions at intervals of 4 weeks. Nine patients were treated with transfected PBMCs; seven patients received all three infusions, one patient received two infusions and one patient received one infusion. Patients suffered from pancreatic, colon or rectal cancer. Drug Product was generally well tolerated, with mild to moderate chills seen in most patients. Dose-limiting toxicity was not observed. No autoimmune toxicity or hypersensitivity reactions were detected.

The results of the IIT study showed that infusions with transfected PBMCs were safe and well tolerated upon single and multiple dosing and no serious effects have been reported. Clinical studies showed that Cbl-b silencing in patient's PBMCs led to enhanced secretion of the inflammatory cytokines IFN-γ and IL-2 following TCR stimulation in vitro. Long-lasting PBMC responses to common tumor antigens were seen over at least 6 months. Efficacy in terms of a reduction in tumor burden has not been shown in clinical studies to date due to the small number of patients enrolled, the advanced stage of cancers that the patients presented with, and the potential for suboptimal dosing with transfected PBMCs as the MTD has not been established.

Four out of 16 patient with single dosing and three out of 11 patients with multiple dosing showed a stabilization of tumor growth (about 30% of patients).

The purpose of a current study is to assess the safety, tolerability, efficacy, and immunological effects of treatment in patients with advanced solid tumors, where well established standard of care medication has ceased to be effective, building on the experience already acquired from the two preceding Phase 1 studies.

This is an open-label, multi-center, dose escalation and expansion study in patients with advanced solid tumors. The study will be performed in two parts. Part A is a Phase 1, dose-finding part in patients in solid tumors. It will evaluate three dose levels of Drug Product using a 3+3 design. Part B is a preliminary dose expansion study at the RP2D with 15 patients for each of the three tumor types (lung cancer, colorectal cancer and head and neck cancer) being treated.

The first part of this study is intended to evaluate the safety, tolerability, and efficacy of transfected PBMCs and to identify the RP2D. Anti-tumor efficacy will also be evaluated. Patients with advanced solid tumors, where well established standard of care medication has ceased to be effective (e.g., progressed on or refractory to at least two prior lines of systemic therapy), will be assigned sequentially to escalating doses of Drug Product using a 3+3 design. Dose escalation requires at least three patients to be treated and observed for at least 3 weeks after the first dose.
Patients with lung cancer, colorectal cancer or head and neck cancer are included in this part of the study.
The incidence of dose limiting toxicity (DLT) will be evaluated during the 3 weeks following the first dose of treatment, but all available safety data will be considered when making decisions about cohort expansion and/or dose escalation.

In dose level 1 three patients receive three doses of 5 × 106 transfected PBMCs/kg at intervals of 3 weeks.

In dose level 2 three patients receive three doses of 1.5 × 107 transfected PBMCs/kg at intervals of 3 weeks.

In dose level 3 three patients receive three doses of 4.5 × 107 transfected PBMCs/kg at intervals of 3 weeks.

In the second part of this study evidence of the desired pharmacodynamic effects should also be apparent in peripheral blood or tumor at the recommended phase 2 dose (RP2D). For example, Drug Product may be considered immunologically active if PBMC IFN-γ production in response to anti-cluster of differentiation (CD)3/CD28 stimulation or to one or more tumor antigens is increased compared with baseline in at least two treated patients.

For each selected tumor type in Part B (expansion phase), 15 patients will be enrolled and treated with Drug Product at the RP2D, i.e., there will be three parallel cohorts of 15 patients (total of 45 patients in Part B).

The Cbl-b silenced PBMCs display a clear increase in proliferation and production of certain cytokines such as interferon gamma (IFN-g) and interleukin (IL)-2 in response to stimulation. Importantly, neither proliferation nor cytokine production is induced in unstimulated T-cells, indicating that silencing Cbl-b enhances T-cell activities only in the context of antigen stimulation. This approach is superior to general systemic activation of all lymphocytes or to the systemic administration of cytokines, which is often associated with severe toxicity and may also have negative immunoregulatory effects. Specifically, Cbl-b deficiency enhances anti-tumor activity of T-cells and NK cells in vitro, resulting in: enhanced expression of inflammatory cytokines and activation markers when stimulated via the T-cell receptor (TCR); enhanced proliferation and anti-tumor cell cytotoxicity; resistance to transforming growth factor β-mediated immune suppression (T-cells); and increased activation of NK cells upon cytokine stimulation or tumor cell contact.
Patients are treated with multiple cycles of GMP-process generated Drug Product at local centers. The adaptive and innate immune system are reactivated after Cbl-b silencing in humans. The effects are amplified with each cycle of treatment by measuring the enrichment of tumor-specific immune cells, the increase of IL-2 level and IFN-γ level, the long-lasting immune responses of PBMCs to common tumor antigens upon TCR stimulation, the memory cell effects of anti-tumor specific immune cells, clinical antitumor activity, and very good safety profile.

### Example 2: Manufacturing of SHP-1 and SHP-2 silenced PBMCs

Similar to Example 1, instead of using siRNA coding for Cbl-b also siRNA coding for SHP-1/PTPN6 and SHP-2/PTPN11 can be used to generate a Drug Product with the method of the invention.

The selected siRNA Oligonucleotides show the following sequences.

### SHP-1/PTPN6

| **SEQ ID NO:** | **strand** | **sequence (5'>3')** |
|---|---|---|
| 61 | sense | GGAACAAAUGCGUCCCAUA |
| 62 | sense | AUACAAACUCCGUACCUUA |
| 63 | sense | UAUGAGAACCUGCACACUA |
| 64 | sense | GCUCCGAUCCCACUAGUGA |

### SHP-2/PTPN11

| **SEQ ID NO:** | | **sequence (5'>3')** |
|---|---|---|
| 65 | sense | GAACAUCACGGGCAAUUAA |
| 66 | sense | GAAGCACAGUACCGAUUUA |
| 67 | sense | GGAGAUGGUUUCACCCAAA |
| 68 | sense | GGACGUUCAUUGUGAUUGA |

### Example 3: Transduction of Leukocytes with large RNAs and DNAs

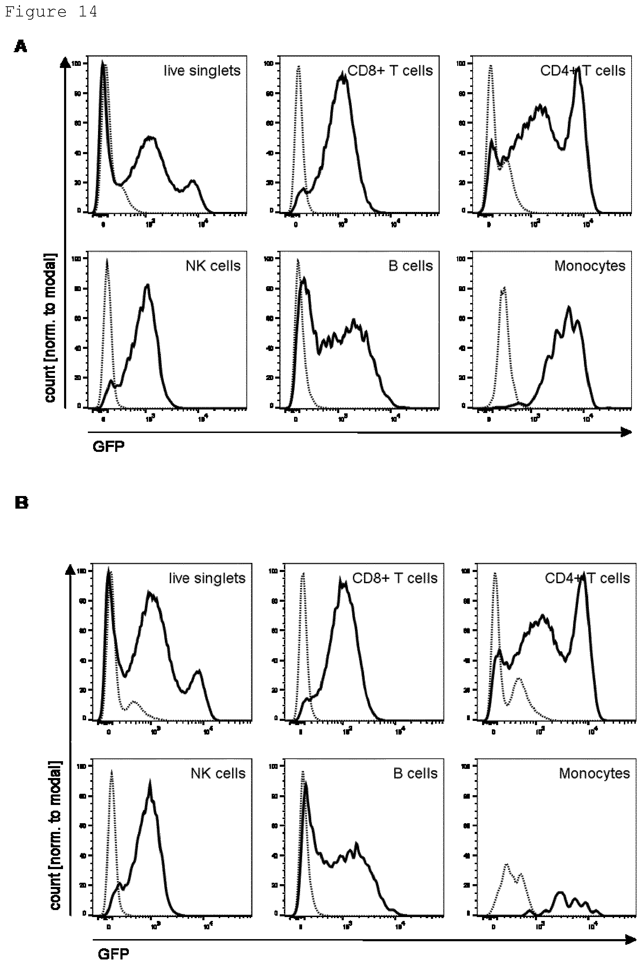

In order to validate the method of the invention for the transduction with nucleic acids that are larger than siRNAs, Leukocytes were purified as described in MAP Step I and electroporated with an mRNA encoding enhanced Green Fluorescent Protein (eGFP, TriLink Biotechnologies) or an GFP encoding DNA plasmid vector using the closed, automated process as described in MAP Step II. After electroporation leukocytes were cultured for up to 48h and analyzed for GFP expression after 24h and 48h by flow cytometry on a Fortessa LSR X-20 (BD Bioscience) as illustrated in Figure 14.

Figure 14 eGFP fluorescence in leukocytes transduced with eGFP-mRNA quantified by flow cytometry after (A) 24h or (B) 48h culture time. Dotted line depicts non-electroporated control, black outline depicts GFP fluorescence in live single cells, CD8+ T cells, CD4+ T cells, NK cells, B cells or CD14+ monocytes.

### List of reference signs

- 1: manufacturing process step I
- 2: manufacturing process step II
- 3: manufacturing process step III
- 4: sample preparation
- 5: sample purification and resuspension
- 6: sample transfection
- 7: immune cells transfected with nucleic acid
- 8: final sample formulation
- 9: obtaining drug product ready for infusion
- 10: control IPC01
- 11: control IPC02
- 12: control S01
- 13: control S02

## Claims

1. An in-vitro or ex-vivo method for modifying immune cells comprising the steps:
(i) providing immune cells from a biological liquid and/or from a resected tumor from a patient, thereby providing a cell sample;
(ii) purifying the immune cells of the cell sample;
(iii) transfecting the purified immune cells with an inhibitory nucleic acid of an immunosuppressive regulator of the immune cells or with a nucleic acid of an immune enhancing factor;
(iv) purifying the transfected immune cells and/or rebuffering the transfected immune cells into a physiological solution;
(v) transferring the transfected immune cells into a container;
wherein the transitions between each two subsequent steps of steps (i) to (v) are in a closed container system.

2. The method of claim 1, wherein immune cells are mononuclear or polymorphonuclear immune cells, preferably peripheral blood mononuclear cells (PBMCs), especially preferred peripheral blood lymphocytes (PBLs), natural killer (NK) cells, T cells, B cells, monocytes, and/or tumor infiltrating lymphocytes (TILs) from solid tumors or from tumor associated fluids.

3. The method of claim 2, wherein the peripheral blood mononuclear cells (PBMCs) are obtained by apheresis from a patient, preferably by a single standard leukapheresis procedure and wherein the tumor infiltrating cells are obtained from processed tumor fragments or from tumor associated liquids.

4. The method of any one of claims 1 to 3, wherein after and/or during purification of the immune cells, red blood cells of the cell sample are lysed with a buffer of a pH value of 7 to 8, preferably with a pH value of 7.4, containing chloride, ammonium, potassium and/or bicarbonate ions, preferably by using an ammonium chloride potassium lysis buffer, and/or platelets in the cell sample are depleted.

5. The method of any one of claims 1 to 4, wherein the transfection is performed by microinjection, liposomes, electroporation, particle gun, magnet-assisted transfection, sonoporation and/or cell squeezing technology of the immune cells.

6. The method of claim 5, wherein transfecting the purified immune cells is performed with at least one nucleic acid, preferably DNA, mRNA or siRNA, and/or PNAs, which causes modification of the gene expression pattern of one or more immune checkpoint inhibitors, chemokine receptors, cytokines, and/or chimeric antigen receptors.

7. The method of any one of claims 1 to 6, wherein transfecting the purified immune cells is performed by electroporation, preferably by flow electroporation or by large volume electroporation, with siRNA for silencing at least one immune checkpoint inhibitor or mRNA encoding immune enhancing factors.

8. The method of claim 6 or 7, wherein said one or more immune checkpoint inhibitor is/are selected from the group comprising Cbl-b, PD-1, PD-L1, PD-L2, CASP, CTLA4, FoxP3, LAG-3, TIM-3, TIGIT, A2AR, KIR, BTLA, VISTA, B7-H3, B7-H4, SHIP, SHP-1, SHP-2, IL-1R8, NKG2A,CD96, CD112R, CD160, CD244, IDO, IRG-1, STAT-3, JAKs, Arg-1, Nos-2, Cish, TGFb, PKA, TNFRSF, or a combination thereof.

9. The method of any one of claims 1 to 8, wherein rebuffering the transfected immune cells comprises rebuffering into a physiological fluid with a stabilizing agent, preferably into 0.9% NaCl solution containing 1% to 4 % autologous human serum, preferably 0.9% NaCl containing 0.7 g/l to 4.5 g/l human serum albumin.

10. The method of any one of claims 1 to 9, wherein in parallel to the rebuffering step immune cells are purified and further depleted from platelets and cell debris, preferably using a spinning-membrane technology, elutriation and/or counter-flow centrifugation.

11. The method of any one of claims 1 to 10, wherein the closed container system comprises at least one container, preferably a bag with immune cells and/or at least one buffer container, and/or at least one tubing set connecting the containers, and/or at least one withdrawal-port, preferably for in-process controls; preferably wherein the container, tubing set and/or the withdrawal port is/are aseptically interconnected, preferably by using a sterile tubing welder.

12. A population of immune cells for use in a method for treating cancer, wherein the population of immune cells is obtained by a method according to at least any of claims 1 to 11.

13. A population of immune cells for use according to claim 12, wherein more than 15 % of immune cells of the biological sample, preferably of NK cells, T cells and B cells contain siRNA, mRNA or DNA after the transfection, preferably electroporation.

14. A population of immune cells for use according to claim 12 or 13, wherein the population of immune cells is administered in at least one dose and one dose comprises 5×10⁶ to 5×10¹⁰, preferably 5×10⁷ to 5×10⁸, transfected PBMCs or TILs per kg body weight of a patient.

15. A population of immune cells for use according to any one of claims 12 to 14, wherein a patient receives intravenously, intratumorally and/or intranodally a single or multiple dosing of population of immune cells, preferably one to ten doses, preferably three to five doses, and doses are administered at intervals of 2 to 10 weeks, preferably 3 to 7 weeks, most preferably at 4 weeks for at least 2.
